# EUROPEAN PATENT SPECIFICATION

(11) **EP 2 494 033 B1**
(45) Date of publication and mention of the grant of the patent: **01.07.2020**
(21) Application number: 10827464.8
(22) Date of filing: 28.10.2010
(51) Int. Cl.: C12N 5/071

(54) **MULTIPOTENT STEM CELLS FROM THE EXTRAHEPATIC BILLARY TREE AND METHODS OF ISOLATING SAME**
MULTIPOTENTE STAMMZELLEN AUS DEM EXTRAHEPATISCHEN BILIÄRTRAKT UND VERFAHREN ZU IHRER ISOLIERUNG
CELLULES SOUCHES PLURIPOTENTES À PARTIR DE L'ARBRE BILIAIRE EXTRAHÉPATIQUE ET PROCÉDÉS POUR LEUR ISOLEMENT

(30) Priority: 30.10.2009 US 256846 P
(43) Date of publication of application: 05.09.2012
(73) Proprietor: The University of North Carolina at Chapel Hill, Chapel Hill, NC 27516 (US); Sapienza Universita di Roma, 00185 Roma (IT)
(72) Inventor: REID, Lola, M., Chapel Hill, NC 27514 (US); WANG, Yunfang, Beijing 27517 (CN); CARDINALE, Vincenzo, 83059 Avellino (IT); GAUDIO, Eugenio, 00197 Rome (IT); CARPINO, Guido, 00199 Rome (IT); ALVARO, Domenico, 00137 Rome (IT); CUI, Cai-Bin, Chapel Hill NC 27516 (US)
(74) Representative: Nederlandsch Octrooibureau
(86) International application number: PCT/US2010/054450
(87) International publication number: WO 2011/053690

(56) References cited:
- US-A- 6 069 005
- US-A1- 2002 182 188
- US-A1- 2003 032 182
- US-A1- 2003 229 045
- US-A1- 2004 018 621
- US-A1- 2007 036 765
- US-A1- 2007 099 297
- US-A1- 2009 092 586
- US-A1- 2009 191 159
- LIU C ET AL: "Presence of markers for liver progenitor cells in human-derived intrahepatic biliary epithelial cells", LIVER INTERNATIONAL, vol. 24, no. 6, 1 December 2004 (2004-12-01), pages 669-678, XP055193223, ISSN: 1478-3223, DOI: 10.1111/j.1478-3231.2004.0971.x
- KUWAHARA R ET AL: "The hepatic stem cell niche: Identification by label-retaining cell assay", HEPATOLOGY, vol. 47, no. 6, 1 June 2008 (2008-06-01), pages 1994-2002, XP055193225, ISSN: 0270-9139, DOI: 10.1002/hep.22218
- MWANGI S ET AL: "W1822 Gdnf-Mediated Increase in beta-Cell Mass and Resistance to STZ-Induced Hyperglycemia Is Associated with Increased Cell Proliferation, and Pdx1 and Sox9 Expression", GASTROENTEROLOGY, vol. 134, no. 4, W1822, 1 April 2008 (2008-04-01), pages A722-A723, XP023435173, ISSN: 0016-5085, DOI: 10.1016/S0016-5085(08)63376-4 [retrieved on 2008-04-01]
- CARDINALE V ET AL: "Multipotent stem cells in the biliary tree.", ITALIAN JOURNAL OF ANATOMY AND EMBRYOLOGY, vol. 115, no. 1-2, 2010, pages 85-90, XP055193335, IT ISSN: 1122-6714, DOI: 10.13128/IJAE-8706
- CARDINALE V ET AL: "Multipotent stem cells reside in human extrahepatic bile ducts and can give rise to hepatocytes, choliangiocytes and pancreatic islets cells", DIGESTIVE AND LIVER DISEASE, vol. 42, no. Suppl. 1, F.N.1, February 2010 (2010-02), page S33, XP026976123, ISSN: 1590-8658, DOI: 10.1016/S1590-8658(10)60455-0 [retrieved on 2010-02-01]
- CARDINALE V ET AL: "Multipotent stem cells reside in human extrahepatic bile ducts and can give rise to hepatocytes, choliangiocytes and pancreatic islets cells", DIGESTIVE AND LIVER DISEASE, vol. 42, no. Suppl. 2, CS.3.2, March 2010 (2010-03), pages S66-S67, XP026975685, ISSN: 1590-8658, DOI: 10.1016/S1590-8658(10)60017-5 [retrieved on 2010-03-01]
- CARDINALE V ET AL: "Multipotent stem cells reside in human extrahepatic bile ducts and can give rise to hepatocytes, choliangiocytes and pancreatic islets cells", JOURNAL OF HEPATOLOGY, vol. 52, no. Suppl. 1, 922, April 2010 (2010-04), page S358, XP026998820, ISSN: 0168-8278, DOI: 10.1016/S0168-8278(10)60923-3
- CARDINALE V ET AL: "Multipotent stem/progenitor cells in human biliary tree give rise to hepatocytes, cholangiocytes, and pancreatic islets", HEPATOLOGY, vol. 54, no. 6, 2 December 2011 (2011-12-02), pages 2159-2172, XP055193235, ISSN: 0270-9139, DOI: 10.1002/hep.24590
- GOLDING ET AL.: 'Reactive Biliary Epithelium: The Product of a Pluripotential Stem Cell Compartment?' HUMAN PATHOLOGY vol. 27, no. 9, September 1996, pages 872 - 884, XP008162693
- ALISON ET AL.: 'Pluripotential liver stem cells: facultative stem cells located in the biliary tree' CELL PROLIF. vol. 29, no. 7, July 1996, pages 373 - 402, XP055093947

## Description

### FIELD OF THE INVENTION

The present invention relates generally to multipotent progenitor cells, including multipotent stem cells, and cell populations comprising such progenitors or stem cells, in the biliary tree, liver and pancreas. More particularly, the present invention relates to multipotent progenitors or stem cells and cell populations comprising such progenitors or stem cells derived from portions of the extrahepatic biliary tree. It includes compositions comprising same and methods of identifying, isolating, maintaining, expanding and differentiating such cells and cell populations *in vitro* and *in vivo.*

### BACKGROUND OF THE INVENTION

Dead, dying, or dysfunctional cells are the cause of many known diseases, including diabetes and Alzheimer's diseases. One method of treating these ailments has focused on transplanting whole organs or parts thereof from donors to replace some or all of the functions of the "diseased" cells. Though often successful in stemming or retarding the course of some diseases, organ transplantation is accompanied by sizable morbidity/mortality, as are all major surgical interventions, and survival of the transplant is dependent on chronic administration of potent, systemic immuno-suppressants that often result in undesirable side effects. No matter the success of these approaches, however, they are inherently limited by the availability of donors.

An alternate approach in "regenerative medicine" is the use of stem cells and progenitors to repopulate whole organs, parts thereof, or at least their functions. Indeed, stem cells can be injected or implanted with relatively minor surgical procedures, often with negligible occurrence of adverse events. Because stem cells are not immunogenic (or are minimally immunogenic), they require relatively little, if any, immunosuppressants for the initial seeding of the cells.

Stem cells are rare cells requiring precise technologies to isolate and well-defined conditions to propagate *in vivo* and *in vitro.* Therefore, there is an urgent need to establish alternative and complementary sources of transplantable, functional stem cells and progenitor cells and tissue for clinical use.

### SUMMARY OF THE INVENTION

Here is disclosed a composition comprising mammalian multipotent stem/progenitor cells capable of differentiating into multiple endodermal lineages (e.g., hepatic lineage, a biliary lineage, a pancreatic lineage, or a combination thereof) is provided, wherein the cells are obtained from biliary tree tissue (*e.g.,* any portion of the biliary tree, including the hilum, common hepatic duct, cystic duct, common duct, common hepato-pancreatic duct gall bladder and peribiliary glands or progenitor cells or stem cells derived from the peribiliary glands) of a mammal, including a human mammal, fetus, neonate, child (pediatric), adult, or a deceased person up to 72 hours post mortem. Compositions comprising a population of cells comprising such mammalian multipotent stem/progenitor cells and/or a population of mammalian cells enriched for such multipotent stem/progenitor cells are also provided.

The multipotent stem/progenitor cells may express: (i) at least one marker indicative of early stage liver cell lineages (*e.g.*, HNF6, HES1, CK19, albumin, or AFP); (ii) at least one marker indicative of early stage pancreatic cell lineages (*e.g.*, PDX1, PROX1, NGN3, or insulin); and at least one marker selected from those in categories (a) - (c): (a) at least one surface marker found on stem/progenitor cells *(e.g.,* CD133 (prominin), CD44H (hyaluronan receptor), N-CAM, CXCR4, or EpCAM); (b) at least one transcription factor indicative of endodermal stem/progenitors (*e.g*., SOX 9, SOX17, or FOXA2), and (c) weak to moderate expression a pluripotency gene (*e.g*., SOX2, NANOG, KLF4, OCT4A or OCT4). In some instances, the multipotent stem/progenitor cells can express one marker from each of categories (i), (ii) and (a) - (c). The expression may be determined by endpoint and quantitative RT-PCR assay and/or by immunohistochemistry of tissue *in vivo,* of freshly isolated cells, or of cultured cells

The multipotent cells may also express at least one of the following: (i) nuclear expression of telomerase protein; (ii) low to moderate levels of expression of pluripotency genes; (iii) nuclear or perinuclear expression of classic endodermal transcription factors (*e.g*., SOX17, SOX 9, FOXA2, HES1, HNF6, PROX1, HNF3B (hepatocyte nuclear factor-3B, FOXA2, SALL4 (Sal-like protein 4), PDX 1, NGN3, or combinations thereof); (iv) expression of endodermal stem/progenitor surface markers; (v) lack of expression or expression of low and variable levels of lineage markers of mature liver (P450-3A4, transferrin, tyrosine aminotransferase (TAT) and high levels of albumin; lineage markers for mature bile duct comprise AE2, CFTR, secretin receptor, aquaporins, or combinations thereof), mature bile duct, or mature endocrine pancreas (*e.g*., insulin, glucagon, somatostatin, amylase or combinations thereof); (vi) lack of expression of markers for mesenchymal cells, endothelial cells or hemopoietic cells.

An embodiment of the invention is a method of obtaining, isolating, and/or identifying a mammalian multipotent stem/progenitor cells capable of differentiating into multiple endodermal lineages is provided, wherein the cells are obtained from biliary tree tissue (*e.g*., the biliary tree tissue comprises hilum, common hepatic duct, cystic duct, common duct, common hepato-pancreatic duct and gallbladder, or combinations thereof) of a mammal, the method comprising obtaining biliary tree tissue, and sequentially, and in any order, or substantially simultaneously obtaining cells that are positive for: (i) at least one stem cell surface marker selected from EpCAM, N-CAM, CXCR4, CD44H, or CD133; (ii) at least one marker indicative of liver cell lineage stages selected from HNF6, HESI, CK19, albumin, or AFP; and (iii) at least one marker indicative of pancreatic cell lineage stages selected from PDX1, PROX1, NGN3, or insulin. A method of identifying and isolating a population of mammalian multipotent cells enriched for the mammalian multipotent stem/progenitors is also disclosed.

According to the method, the basal medium may be any basal medium rich in nutrients and with no copper and low calcium (below 0.5 mM), preferably supplemented with insulin, transferrin/Fe, selenium, zinc, free fatty acids bound to serum albumin and, optionally, high density lipoprotein. An example of a basal medium is RPMI 1640. The cells may optionally be cultured on plastic alone or plastic coated with collagen IV, collagen III, laminin, hyaluronans, other matrix components from embryonic, fetal, neonatal tissues or combinations thereof. The cells are cultured for at least 24 hours and preferably 7-21 days. According to the method, the isolated cells are 80%, 90%, 95%, 95%, preferably 100% enriched for the stem cells of the present invention. The isolation may be carried out via immunoselection technology (*e.g*., panning, magnetic bead selection, flow cytometry, or combinations thereof) and/or selective culture conditions.

It is also disclosed a method of propagating and/or expanding the novel mammalian multipotent stem/progenitor cells, or a population comprising such cells or enriched for such cells or a partner cell thereof (*e.g*., mesenchymal cells, angioblasts and stellate cell precursors), comprising: culturing the cells on plastic or hyaluronans, or optionally on plastic coated with type III or IV collagen or hyaluronan or other matrix component derived from fetal, neonatal, or embryonic tissue and in a basal medium containing no copper, low calcium (<0.5 mM), insulin, transferrin/Fe, a mixture of free fatty acids bound to serum albumin, and optionally, high density lipoprotein.

A method of lineage restricting the novel multipotent stem/progenitor cells to adult liver cell fates is provided. The method comprises (a) obtaining a cell suspension comprising mammalian multipotent stem/progenitor cells capable of differentiating into multiple endodermal lineages, wherein the cells are obtained from biliary tree tissue of a mammal; (b) embedding the cell suspension into a hydrogel comprising hyaluronan or hyaluronan combined with other matrix components (*e.g*., type IV collagen, laminin, or both); and (c) culturing the cell suspension in basal medium supplemented with copper, calcium (≥0.5 mM), insulin, transferrin/Fe, bFGF, hydrocortisone, glucagon, galactose, tri-iodothyroxine (T3), epidermal growth factor (EGF), hepatocyte growth factor (HGF), high density lipoprotein, and a mixture of free fatty acids bound to albumin, for a time sufficient to allow their differentiation into liver cells.

A method of lineage restricting the novel multipotent stem/progenitor cells to adult pancreatic cell fates is provided. The method comprises (a) obtaining a cell suspension comprising the mammalian multipotent stem/progenitor cells mammalian multipotent stem/progenitor cells capable of differentiating into multiple endodermal lineages, wherein the cells are obtained from biliary tree tissue of a mammal; (b) embedding the cell suspension into a hydrogel comprising hyaluronans or hyaluronans combined with other matrix components; and (c) culturing the cells in a basal medium supplemented with copper, calcium (≥0.5mM), B27, ascorbic acid, insulin, transferrin/Fe, bFGF, cyclopamine, retinoic acid, exendin 4, high density lipoprotein, and a mixture of free fatty acids bound to albumin; and lacking hydrocortisone for a time sufficient to allow their differentiation into pancreatic cells.

A method of lineage restricting the novel multipotent stem/progenitor cells to adult biliary cell fates is provided. The method comprises (a) obtaining a cell suspension comprising the mammalian multipotent stem/progenitor cells mammalian multipotent stem/progenitor cells capable of differentiating into multiple endodermal lineages, wherein the cells are obtained from biliary tree tissue of a mammal; (b) embedding the cell suspension into a hydrogel comprising hyaluronans or hyaluronans in combination with other matrix components (*e.g*., type I collagen); and (c) culturing the cells in a basal medium supplemented with copper, calcium (≥0.5 mM), insulin, transferrin/Fe, hydrocortisone, bFGF, vascular endothelial cell growth factor (VEGF), hepatocyte growth factor (HGF), high density lipoprotein, and a mixture of free fatty acids bound to albumin, for a time sufficient for their differentiation into cholangiocytes.

A method of differentiating cells *in vivo* is provided, comprising transplanting the mammalian multipotent stem/progenitor cells, or populations comprising such cells or enriched for such cells, *in vivo* as cell suspensions or as implants or grafts, with or without prior lineage restriction under appropriate culture conditions, into the liver where they differentiate to liver tissue.

A method of differentiating cells *in vivo* is provided, comprising transplanting the mammalian multipotent stem/progenitor cells of Claim 1, or populations comprising such cells or enriched for such cells, *in vivo* as cell suspensions or as implants or grafts, with or without prior lineage restriction under appropriate culture conditions, into the bile duct where they differentiate into biliary tree tissue.

A method of differentiating cells *in vivo* is provided, comprising transplanting the mammalian multipotent stem/progenitor cells of Claim 1, populations comprising such cells or enriched for such cells, *in vivo* as cell suspensions or as implants or grafts, with or without prior lineage restriction under appropriate culture conditions into the pancreas, under the kidney capsule or into the epididymal fat pads, where they differentiate into functional pancreatic tissue.

### BRIEF DESCRIPTION OF THE DRAWINGS

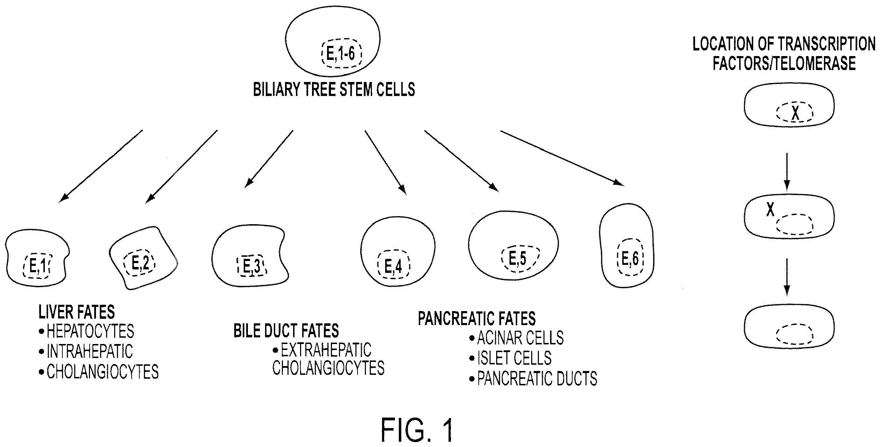

**Figure 1** is a schematic diagram depicting a multipotent biliary stem cell of the present invention having the capacity to differentiate into several types of endodermal tissues. All of the cells express "endodermal" (E) transcription factors (e.g. SOX 9, SOX 17, FOXA2) and other markers of stem/progenitor populations (surface antigens such as CD133, CD44H, EpCAM, CXCR4, NCAM). Lineage restriction results in various adult fates including liver, pancreas, and biliary tree.

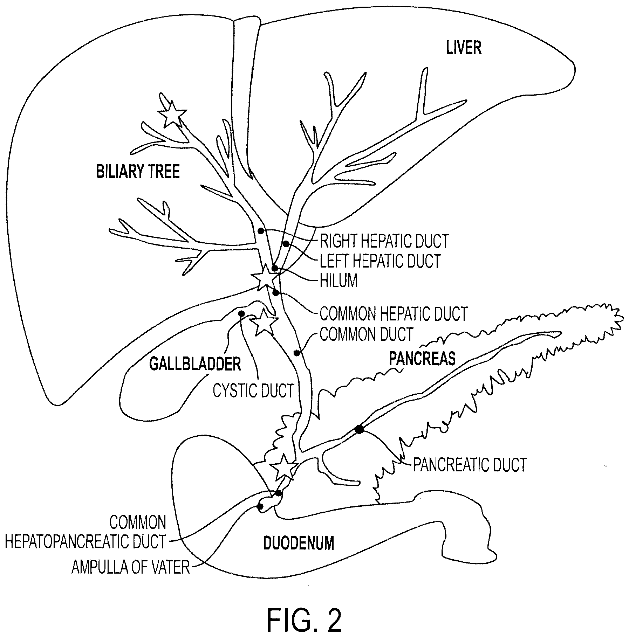

**Figure 2** is a schematic diagram of the biliary tree showing its connections with liver, pancreas and duodenum. Sites at which high numbers of peribiliary glands, the stem cell niches of the biliary tree, may be found are indicated by a star.

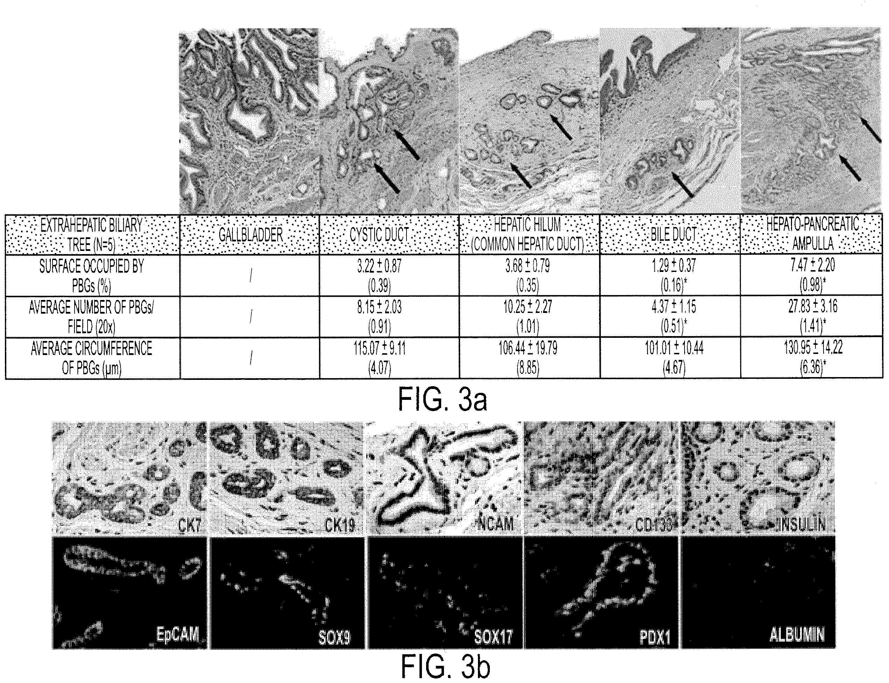

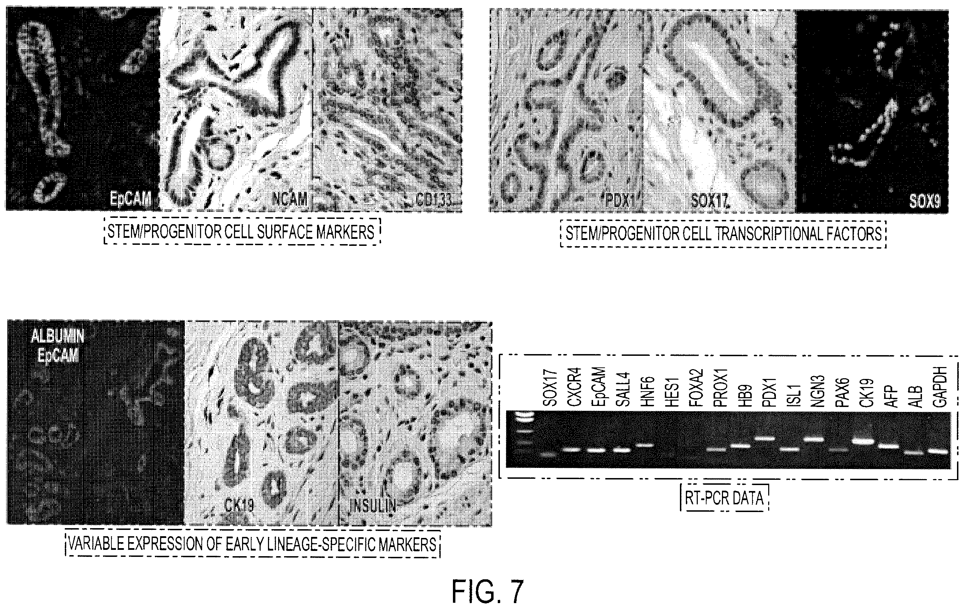

**Figure 3** is a composite of images of histology and immunohistochemistry of different regions of the biliary tree and that were used to identify markers and also the numbers and sizes of peribiliary glands throughout the biliary tree. (a) Distribution and characterization of peribiliary glands (PBGs) in the extrahepatic biliary tree. PBGs are present in the duct walls of the biliary tree and are sites for biliary tree stem/progenitors. A survey was done (n= 5 human biliary trees examined) of the numbers and sizes of the peribiliary glands at varying sites within the biliary tree, of marker profiles of the PBGs. The density of PBGs, expressed as surface occupied by PBGs acini/total area as evaluated by imaging analysis; the number and circumference were histologically analyzed in the different sites of the human extrahepatic biliary tree. The hepato-pancreatic ampulla showed the highest density and number of PBGs; roughly equal numbers were found in the cystic duct and hilum; less were found in bile duct; and none in gallbladder. *p< 0.01. Original magnification x10. **b**: Immunohistochemistry of PBGs *in situ.* The PBGs are positive for CK7, CK19, NCAM, CD133, insulin, EpCAM, SOX9, SOX 17 and PDX1 but negative (or very low levels) for albumin. Variation in these early lineage markers (e.g. albumin) are seen among various peribiliary glands (see **Figure 7** for evidence with respect to albumin). Original magnification x 40.

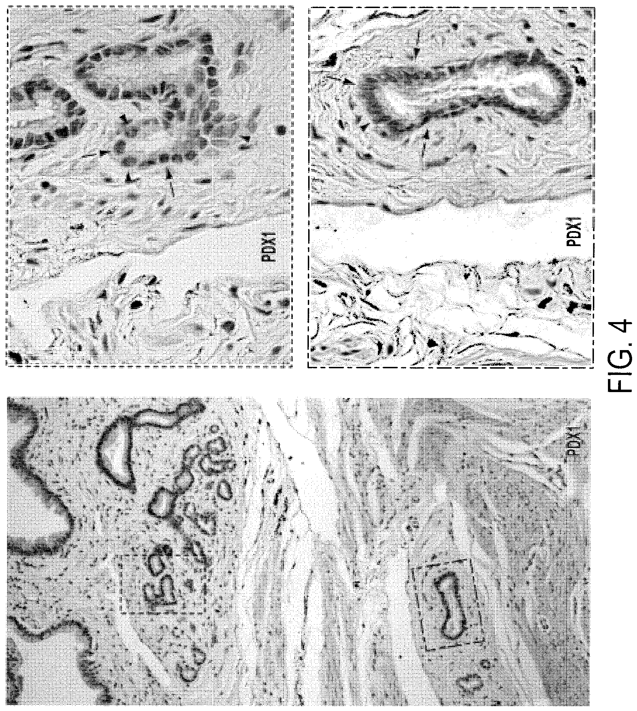

**Figure 4** shows that the stem cells are located primarily within the peribiliary glands (PBGs). Note that there is variability within a single peribiliary gland in the expression of the stem cell markers, which in this case is PDX1. The red arrows indicate the nuclei of cells expressing the transcription factor; the black arrows indicate ones not expressing it.

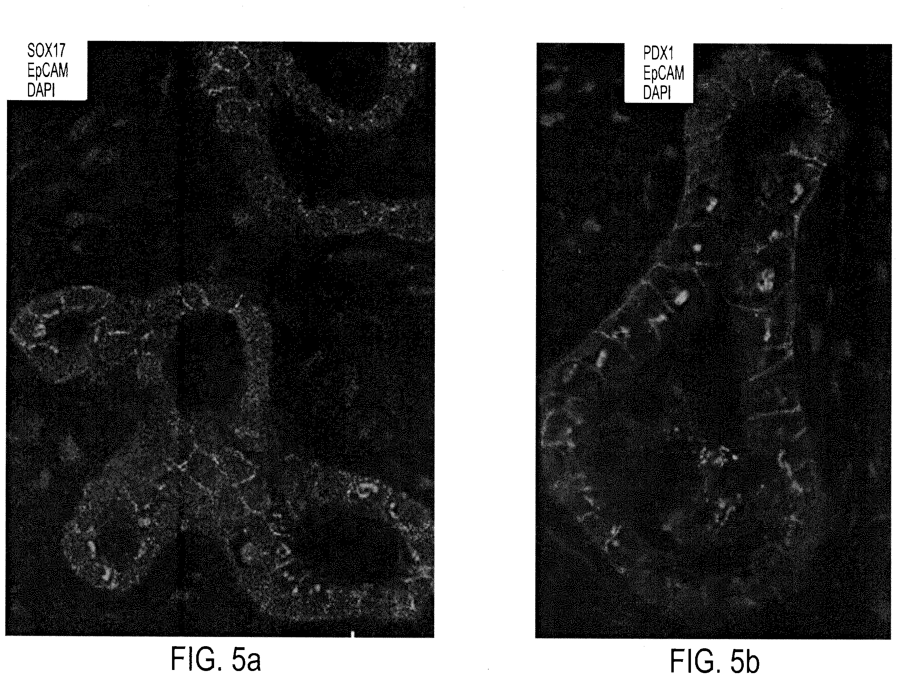

**Figure 5** shows SOX17 (A) and PDX1 (B) along with EpCAM (green) and nuclei stained blue with 4',6-diamidino-2-phenylindole (DAPI) in peribiliary glands of cystic duct.

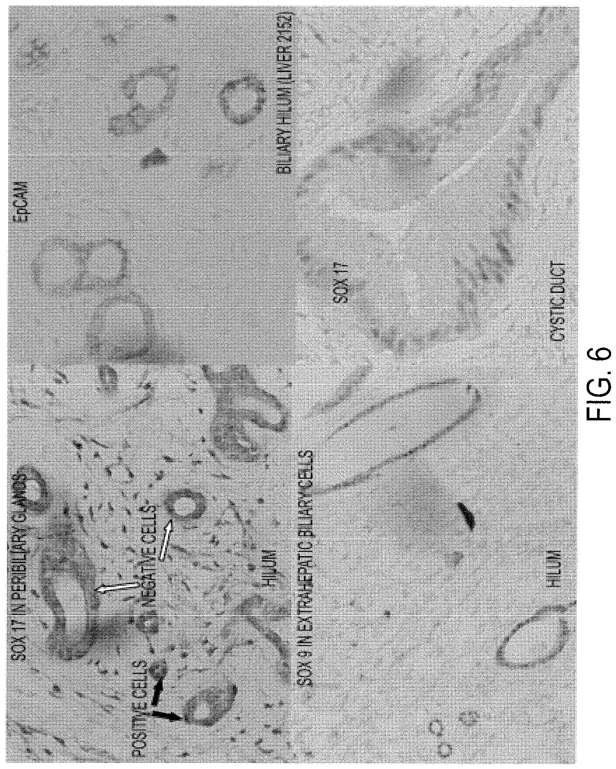

**Figure 6** provides immunohistochemistry data on human biliary tissue and showing expression of EpCAM , SOX 9, and SOX17 in peribiliary glands in cystic duct and hilum.

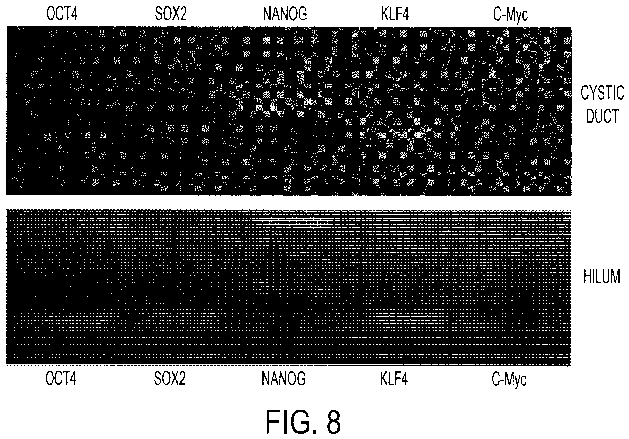

**Figure 7** provides a summary of the key properties of the multipotent Biliary Tree Stem Cells. These include surface markers of stem/progenitors; transcription factors typical of endodermal progenitors; variable expression of early lineage stage markers of liver, biliary tree, and pancreas; and in **Figure 8** the weak to moderate expression of pluripotency genes. Representative RT-PCR assays for hepatic hilum indicate a broad repertoire of endodermal transcription factors (SOX 9, SOX 17, FOXA2, PDX1, NGN3, etc.) and classic stem/progenitor surface markers (e.g. EpCAM, NCAM, CXCR4, CD133).
**Figure 8** shows RT-PCR data indicating expression of pluripotency genes by cystic duct and hilum tissue. Their weak to moderate expression provides additional evidence for the ability of the Biliary Tree Stem Cells to self-replicate. Pluipotency genes are ones found expressed in embryonic stem (ES) cells and ones able to yield induced pluripotent stem (iPS) cells if varying combinations of these genes are transfected into somatic cells. At least 5 genes have been identified: OCT 4, SOX2, NANOG, KLF4 and c-MYC. Weak to moderate levels of expression occur in biliary tree tissue and in isolated Biliary Tree Stem Cells for OCT 4, SOX2, NANOG, KLF4, but not c-MYC.

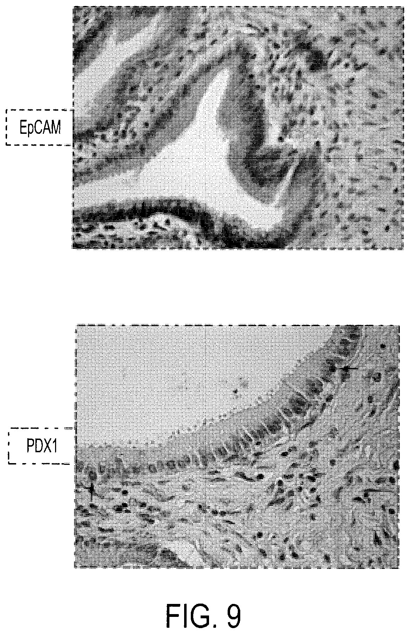

**Figure 9****.** Gallbladder does not contain peribiliary glands (as shown also in **Figure** 3). However, cells expressing some of the stem/progenitor transcription factors and surface markers are present in gallbladder cells. Note that the brownish stain indicative of expression of the marker assayed (EpCAM or PDX1) is present in the cells at the surface of the gallbladder.

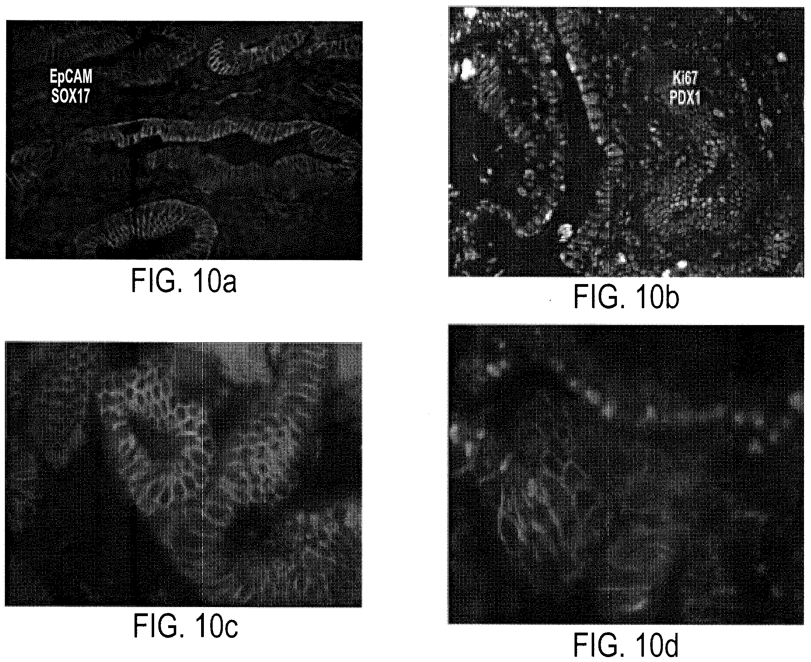

**Figure 10** shows immunohistochemistry data on gallbladder tissue that has no peribiliary glands. **(a,c,d).** The cells are positive for EpCAM (green) and have perinuclear staining for endodermal transcription factors (shown is SOX 17-red). (b) the cells also express PDX1 (pink) and are markedly proliferative as indicated by Ki67 staining (green).

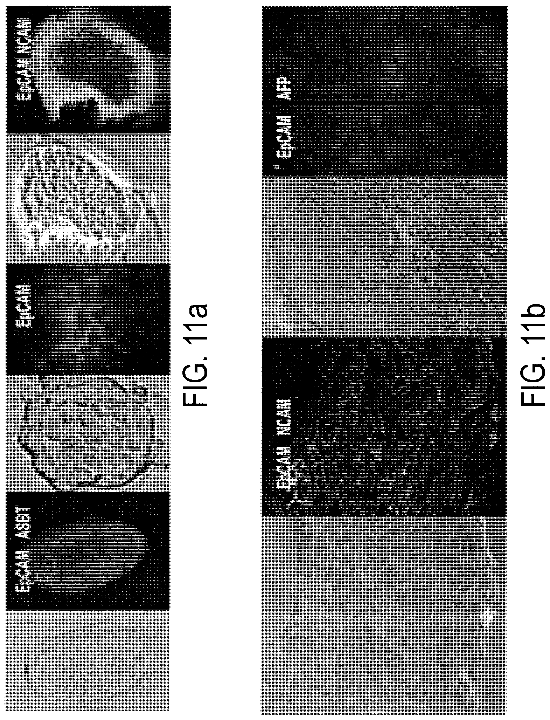

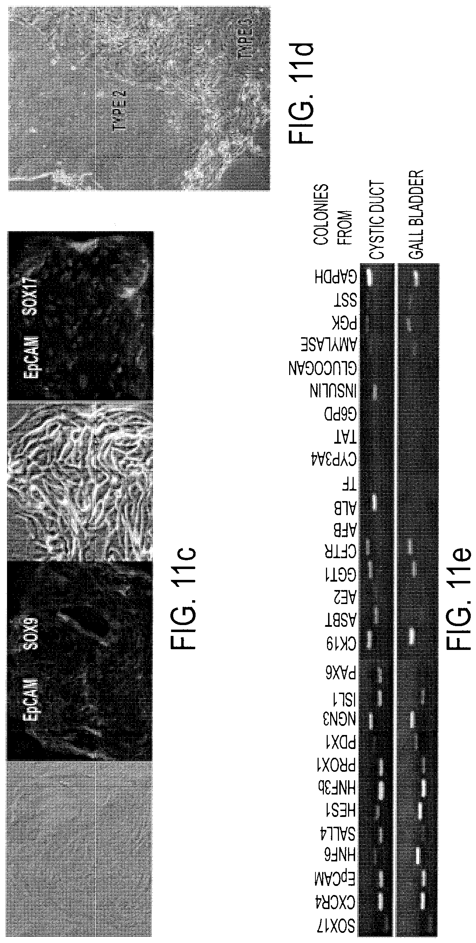

**Figure 11****.** Stem/progenitor cell colonies from biliary tree tissue. Cells were isolated and cultured in serum-free Kubota's Medium and on culture plastic. Three distinct colony types, Types 1-3, were observed under these conditions that are not permissive for mature cells but rather select for endodermal stem/progenitors. Immunohistochemical staining indicates expression for specific genes indicated by text in the color indicated for the marker. All sections are stained with 4',6-diamidino-2-phenylindole (DAPI) providing a blue color indicating nuclei. *Colony type 1* (a) aggregates of cells strongly expressing EpCAM, NCAM, and ASBT. The cells grew slowly (with divisions every 3-4 days or longer). *Colony type 2 cells* (b) with a phenotype closely similar to that of hHpSCs and with 100% of the cells expressing EpCAM, NCAM but none expressing AFP (with divisions every 36-40 hours). *Colony type 3* (c) are comprised of undulating, swirling cells with EpCAM expression at the edges but not interiors of the colonies and with high levels of expression of SOX 17, PDX1, or SOX 9 in the interior cells (with divisions every 36-40 hours). The magnification for all of the images is 20X. Pictures are representative of findings from more than 10 experiments in which the colonies were monitored for 4-8 weeks.
   Evidence of a relationship between cells of colony types 2 and 3 is shown in a low magnification (4X) image (d). It is unknown if one is a precursor of the other. RT-PCR assays (e) indicate the expression of diverse genes in the colonies from cystic duct versus from gall bladder. The RT-PCR assays from the two tissues are quite similar, but those from cystic duct have weak expression of albumin and insulin, two genes not expressed by progenitor cells which were obtained from the gallbladder and growing in serum-free Kubota's Medium and on plastic.

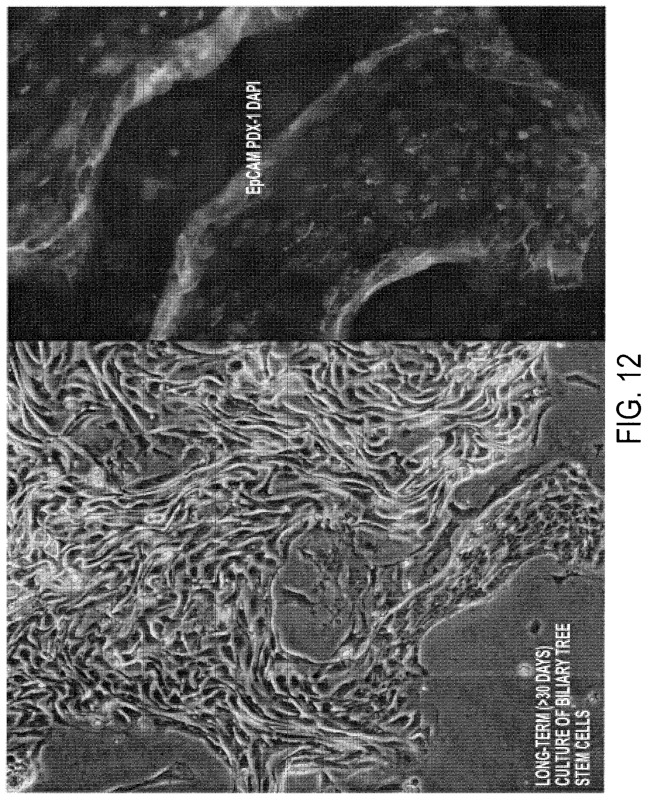

**Figure 12****.** The Biliary Tree Stem Cells, especially those of type 2 and 3 colonies, expand steadily for months resulting in colonies of cells with stable expression of stem/progenitor markers. This is partial evidence for self-replication of the cells; additional evidence is provided by the expression of pluripotency genes **(****Figure 8****).** Here is shown a type 3 colony after a month in culture with maintenance of expression of PDX1 (orange/red) in the cells that are at the center of the colony and with EpCAM (green) expressed only at the edges of the colony, sites at which there is slight differentiation. DAPI staining (blue) indicates the nuclei of the cells.

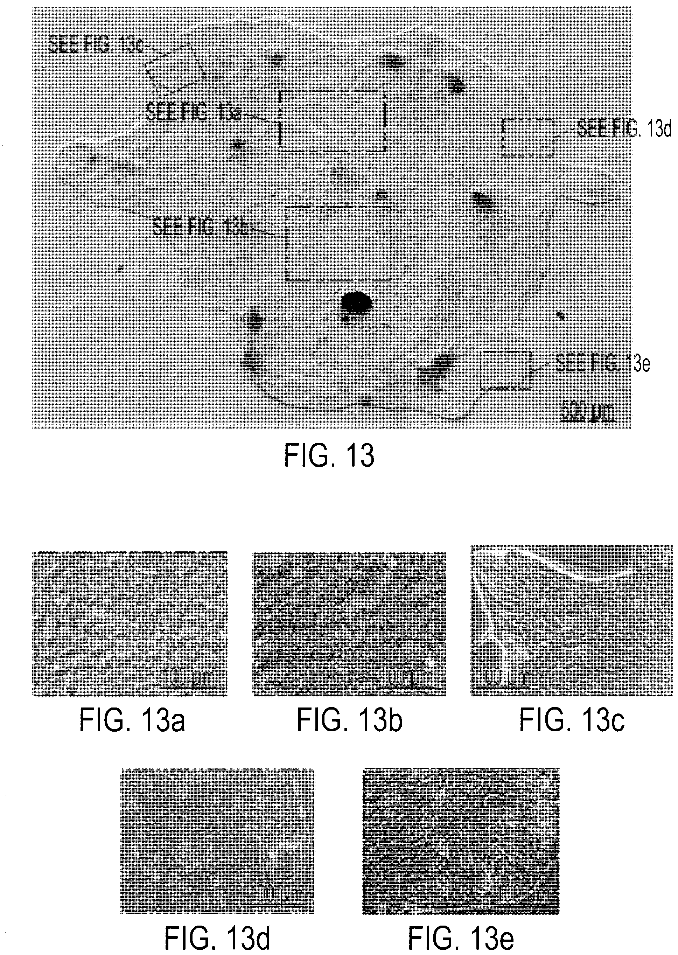

**Figure 13****.** Phase micrograph of a biliary tree stem cell colony in culture for more than 8 weeks on culture plastic and in serum-free Kubota's Medium. The colony was initiated from 1-2 cells derived from adult human biliary tree tissue. To estimate the number of cells within the colony, magnified images were prepared from multiple regions of the colony (representative ones are indicated in the rectangular areas demarcated by colored outlines) and these areas imaged with Metamorph software and used to obtain cell numbers. The sampling of regions resulted in estimates of more than 500,000 cells in the colony. From fetal biliary tree tissue, we observe routinely >50 such colonies; from cystic duct and hilum tissue from adults, we routinely obtain >100 such colonies.

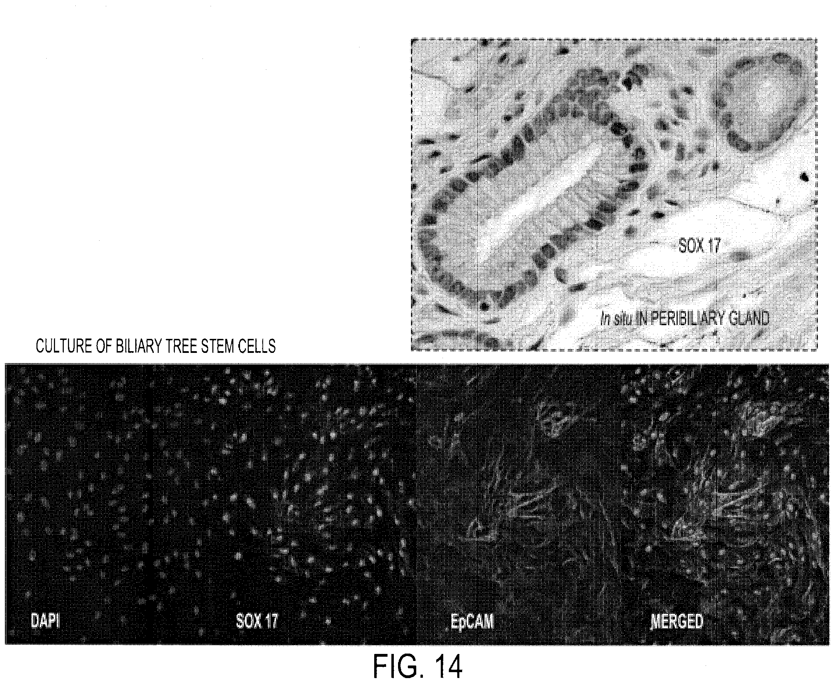

**Figure 14****.** Location of transcription factors (or of telomerase protein) in tissue (*in situ)* or in cultures of Biliary Tree Stem Cells. Immunohistochemistry revealed that the transcription factors and telomerase protein were present intranuclearly in some cells and perinuclearly or cytoplasmically in others. The significance of this is not known, though we hypothesize that nuclear localization implicates an active factor and perinuclear or cytoplasmic location might be a storage form. In these images, SOX 17 is expressed within the nucleus of some, but not all, of the cells of a peribiliary gland in vivo (*in situ* data) and is expressed within the nucleus of all of the Biliary Tree Stem Cells in culture on plastic and in Kubota's Medium. In the images of the cultures, the nuclei are blue from staining with DAPI; some of the cells express EpCAM (green); but all have intranuclear localization of SOX 17 (red). When the images are merged, the nuclei appear pink in color (merged blue and red colors).

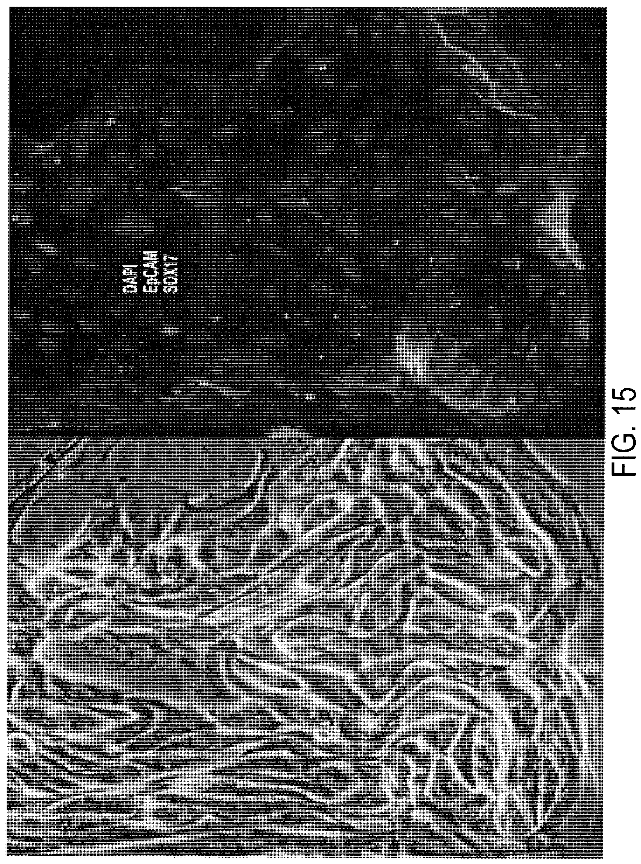

**Figure 15****.** Type 3 colony of Biliary Tree Stem Cells demonstrating both nuclear and perinuclear localization of the transcription factor, SOX 17 (red/pink). The cells at the perimeter of the colony are positive for EpCAM (green). The nuclei are stained blue with DAPI

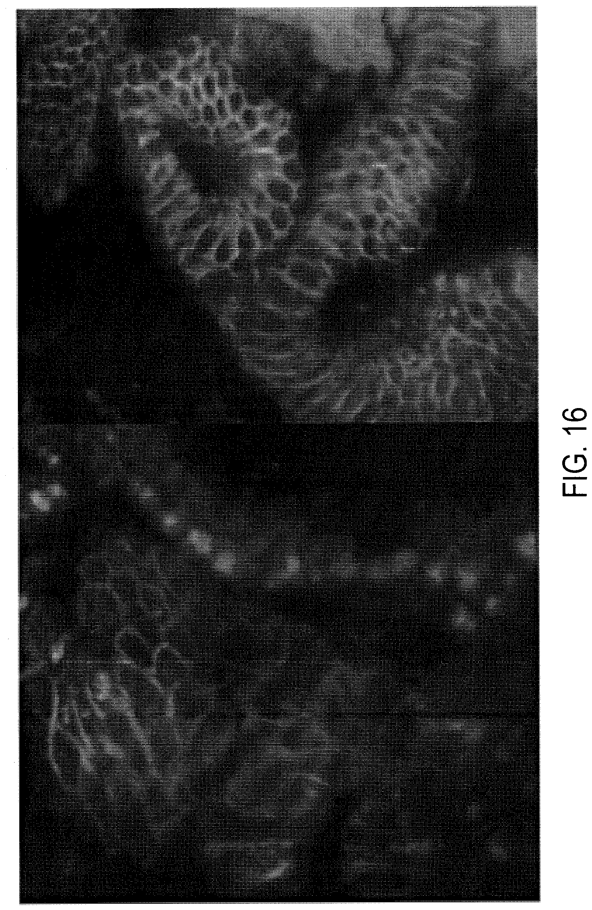

**Figure 16****.** *In situ* staining of gallbladder cells demonstrating only perinuclear and cytoplasmic staining of SOX 17 (red/orange). The cell membranes are positive for EpCAM (green), and the nuclei are stained blue with DAPI.

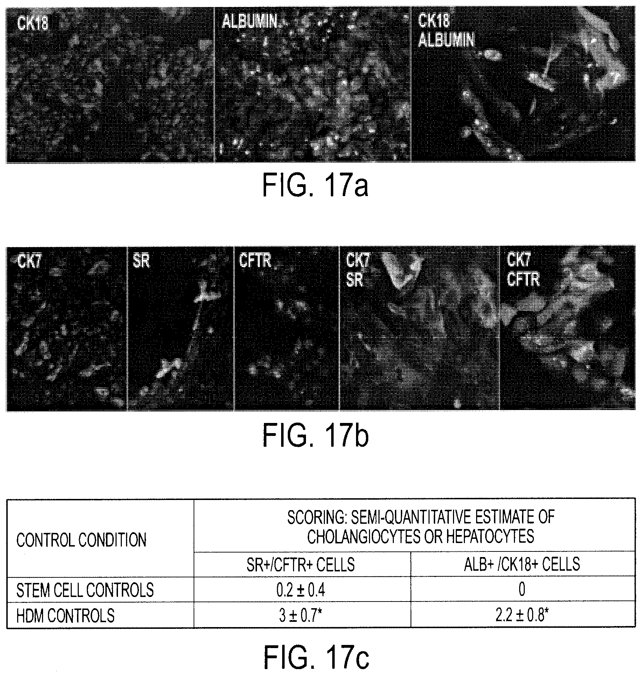

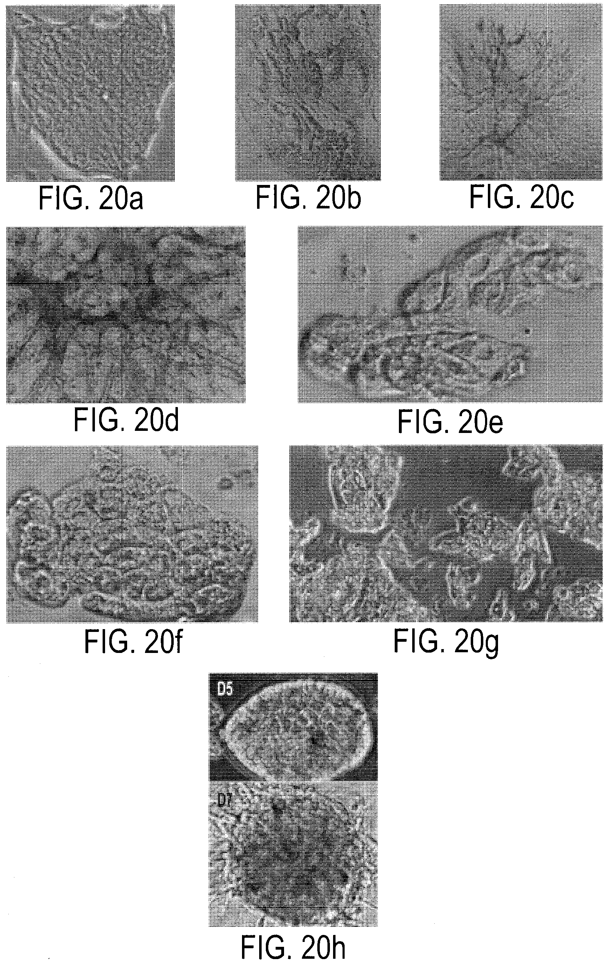

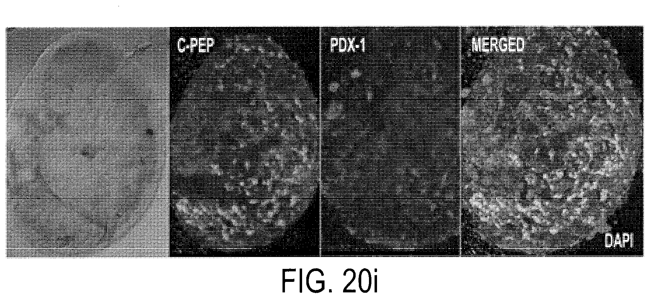

**Figure 17****.** Multipotentiality of the Biliary Tree Stem Cells. Effects of Hormonally Defined Media (HDM) alone on differentiation to an hepatocytic or cholangiocytic fate. The stem cells remain indefinitely as stem/progenitors if cultured on plastic and in Kubota's Medium. They will differentiate towards an adult fate if the medium is changed to a hormonally defined medium (HDM) tailored for an adult fate. They will differentiate faster and more efficiently to an adult fate if the HDM is used in combination with plating the cells on or embedding them into forms of extracellular matrix **(****Figures 20-22****).** In these studies are shown representative data indicating the effect of a specific HDM on the differentiation of the biliary tree stem/progenitors towards an hepatocytic (HDM-L) versus cholangiocytic (HDM-C) fate.
   Biliary tree stem/progenitors from colonies maintained under self-replication conditions (culture plastic and serum-free Kubota's Medium or its equivalent) were transferred either in HDM tailored for either hepatocytes (HDM-L), the upper row of images, or for cholangiocytes (HDM-C), the lower panel of images. **HDM-L Effects.:** Immunofluorescence (a) after 7 days. Cells were diffusely positive for CK18 (red) and/or albumin (blue-green). Magnification: 20X. Semi-quantitative data (c) comparing the numbers of hepatocytes (CK18+/albumin+cells) under self-replication conditions (score: 0) versus in HDM-L (score: 2.2 ± 0.8), findings that translate to >20% of the cells having lineage restricted to an hepatocytic fate. **HDM-C effects:.** Immunofluorescence (b) after 7 days. Cells were diffusely positive for CK7, secretin receptor (SR), and CFTR. They co-expressed CK7/SR and CK7/CFTR. Original Magnifications are 20X except for c4 and c5 that are 40X. Semi-quantitative estimate (c) of % of SR+/CFTR+ cells in the conditions for self-replication *(0*.*2* ± 0.4;<5% of the cells) versus in the HDM-C (3 ± 0.7 which equates to over 30 % of the cells lineage restricting to cholangiocytes.

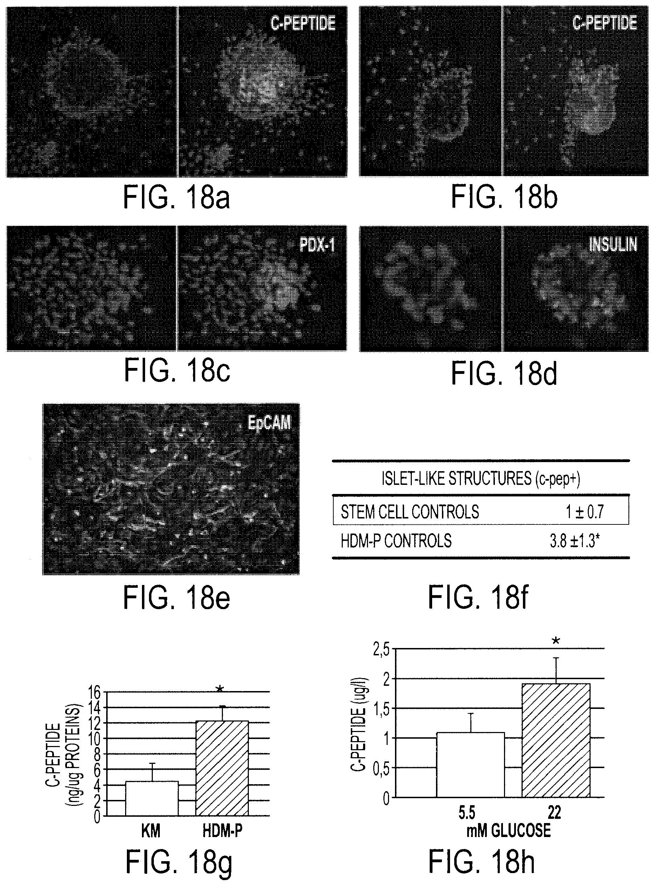

**Figure 18****.** Multipotentiality of the Biliary Tree Stem Cells. Effects of Hormonally Defined Medium tailored to pancreas (HDM-P) alone on differentiation to a pancreatic fate. **HDM-P Effects:** Immunofluorescence for pancreatic markers after 7 days of culture in HDM-P. At the periphery of the colonies, cell aggregation and condensation occurred to form Islet-like structures **(a-d)** containing c-peptide **(a,b),** PDX-1 **(c)** and insulin **(d).** Undifferentiated cells (EpCAM+ cells) were found within the colony centers **(e).** Original Magnifications: 20X. The number of islet-like structures **(f)** found in the conditions for self-replication (1 ±0.7; <10% of the cells) were much lower than that in the HDM-P (3.8 ±1.3; ∼40% of the cells). The levels of C-peptide **(g)** in ng/*µ*g of protein under glucose concentrations found in all RPMI 1640 formulations (11.1 mM) for a 2 hour incubation in the conditions for self-replication was 4.5 ± 2.25 versus 12.3 ± 1.9 ng/ *µ*g in the HDM-P . The data are expressed as Means ± Standard Error, N=4; *p < 0.05. (**h**) Glucose stimulated c-peptide secretion was observed in the HDM-P controls with the levels of the c-peptide in the medium, in *µ*g/l, at 1.10 ± 0.32 ug/l in low levels of glucose (5.5 mM) to 1.92 ± 0.43 ug/l in high glucose (22 mM) n=7; *p < 0.01.

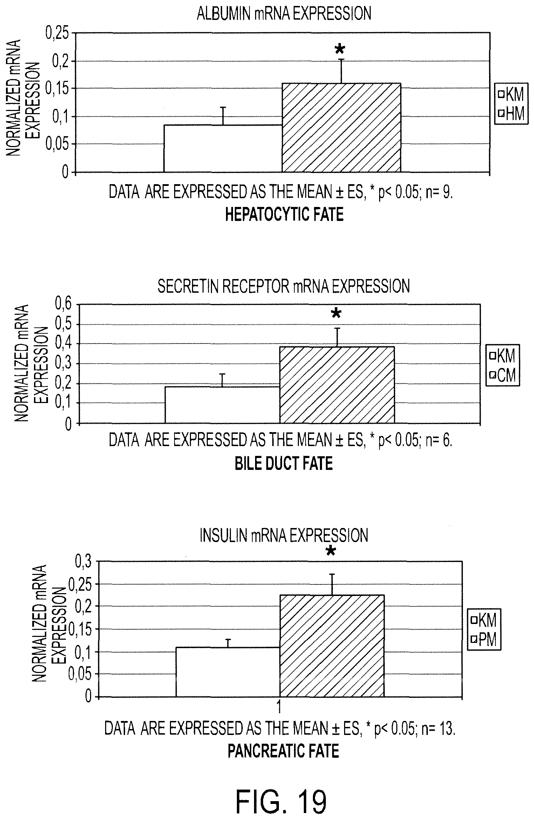

**Figure 19****.** Multipotentiality of the Biliary Tree Stem Cells. Quantitative (q)-RT-PCR analyses for effects of medium alone. The assays were done on biliary tree stem cell cultures maintained under self-replication conditions (culture plastic and Kubota's Medium) versus in a serum-free, hormonally defined medium (HDM -L or HDM-C or HDM-P) alone tailored for hepatocytes, cholangiocytes versus pancreatic cells. The mRNA relative expression levels were calculated by ranking the normalized ΔCt values against the geometric mean of the three most stable housekeeping genes. The data are indicated plus or minus (±) standard deviation. The significance levels for each of these was *p<0.05. n= the number of experiment done.
**Figure 20****.** Multipotentiality of the Biliary Tree Stem Cells. Effects of both an HDM and extracellular matrix components on the differentiation of the Biliary Tree Stem Cells. The biliary stem cells were cultured on plastic and in Kubota's Medium or its equivalent (self-replication conditions) for over a month resulting in colonies such as the one in (a). Such a representative colony was dispersed, and the cells were transferred to one of 3 differentiation conditions, presented in a 3-dimensional (3-D) format and comprised of an HDM + matrix components tailored for the desired adult fate: one for cholangiocytes (b), hepatocytes (c), or for pancreatic *β*-cells (d) They were maintained for up to 9 days in culture and then assayed for tissue-specific fates which indicated that these Biliary Tree Stem Cells can lineage restrict to multiple adult fates (here provided the examples of cholangiocytes, hepatocytes or pancreatic *β*-cells) depending on the culture conditions. Row 3=dithizone (DTZ) staining. (indication of pancreatic *α* and *β* cells).D=days in culture; Glu=glucagon; C-P= C-peptide, indicative of insulin secretion

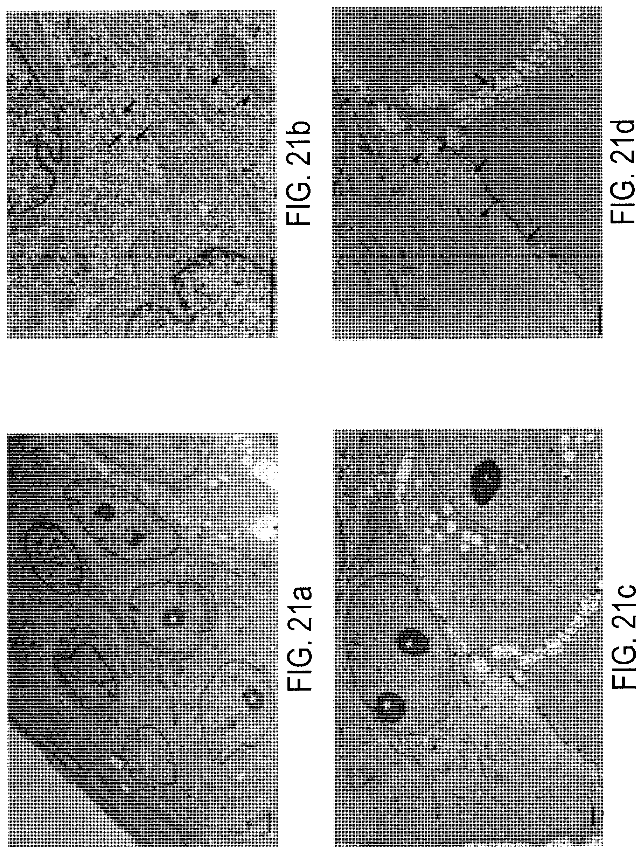

**Figure 21****.** Transmission electron microscopy of Biliary Tree Stem Cells under self replication conditions (a-b) vs ones in the 3-D differentiation conditions to yield mature liver cells (c-d).Large and polygonal cells were present and cell nuclei display one or more nucleoli. (c) Adjacent cells form well-defined bile canaliculi (*arrows).* Bile canaliculi are closed by junctional complexes *(arrowheads).* Few microvilli are present in the lumen. (d). Thus, the cells were able to mature to adult hepatocytes and intrahepatic cholangiocytes. Bar=2*µ*m

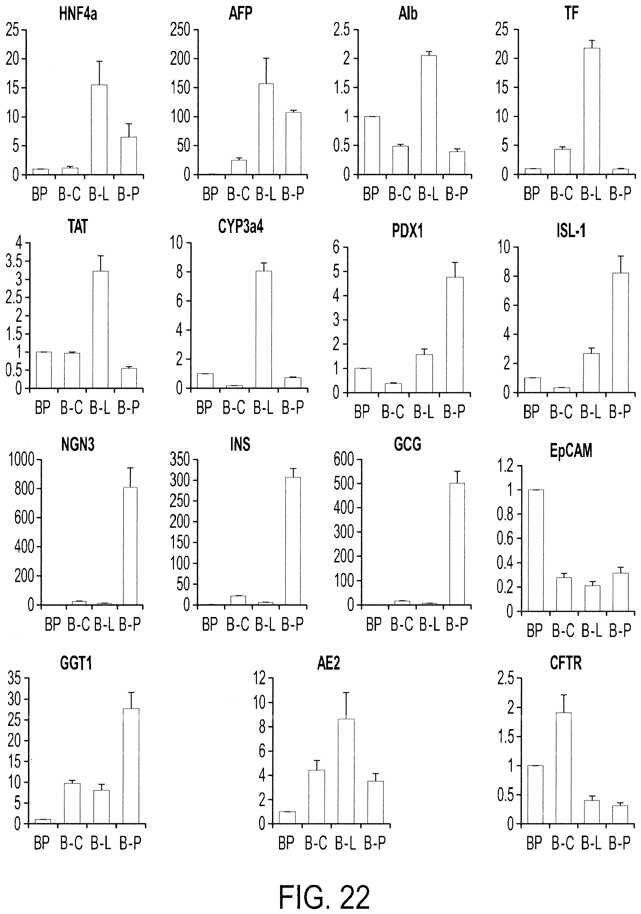

**Figure 22****.** Proof of multipotentiality of the biliary tree stem/progenitors as given by by quantitative (q)-RT-PCR analyses on cultures under self-replication versus 3-D differentiation conditions. **a**. The biliary tree stem/progenitors were cultured in Kubota's Medium and on plastic for 2 months (self-replication conditions). The colonies were either maintained in those conditions and thereby yielding biliary stem/progenitor cells (BP) or transferred into the *3-D* Differentiation conditions for hepatocytes (B-L), cholangiocytes (B-C) or pancreatic islets (B-P), and all were cultured for an additional 2 weeks. The cultures were then prepared for qRT-PCR analyses of genes relevant to each of the adult fates. The data were prepared as histograms in which the levels of expression of the genes in the self-replication conditions (BP) were given the value of 1.0, and the value of each gene in cells after differentiation to an adult fate was given as the fold change relative to that in BPs. The asterisk on some of the histograms indicates those of statistical significance (p<0.01 or p<0.001). N=2, each experiment with triplicate samples. **BP** = biliary stem/progenitors; **B-C** = cells lineage restricted to bile duct (cholangiocytes); **B-L** = cells lineage restricted to liver (hepatocytes); **B-P** = cells lineage restricted to pancreas (islets). The genes assayed were **GGT1** = gamma glutamyl transpeptidase-1; **AE2** = anion exchanger 2; **CFTR** = cystic fibrosis transmembrane conductance regulator; **HNF4a** = hepatocyte nuclear factor 4A; **AFP** = alpha-fetoprotein; **ALB=** albumin; **TF=** transferrin; **TAT** = tyrosine aminotransferase; **CYP3A4** = cytochrome P450 3A4; **pdx1=** Pancreatic and duodenal homeobox 1; **ISL-1** = ISL LIM homeobox 1; **NGN3** = neurogenin 3; **INS** = insulin; **GCG** = glucagon.

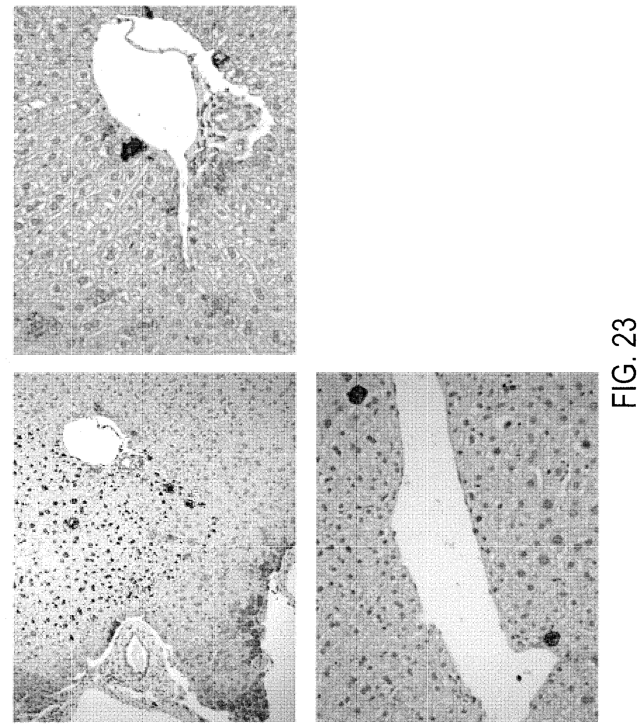

**Figure 23****.** Multipotentiality of the Biliary Tree Stem Cells as indicated by *in vivo* studies: Hepatic Fate. Biliary tree stem cells were maintained in culture under self-replication conditions (Kubota's Medium or its equivalent and culture plastic) and then were injected into the livers of immunocompromised, adult mice with quiescent livers (that is, no liver injury was induced). Liver sections prepared from the mice were then analyzed by immunohistochemistry for human specific markers indicative of hepatocytes. In this image, the section was stained for Dako's Anti human Hepar-1. The sections revealed that one can identify 6.52 ± 2.5% of the total area occupied by human hepatocytes positive for human Hepar-1, a classic hepatocyte marker.

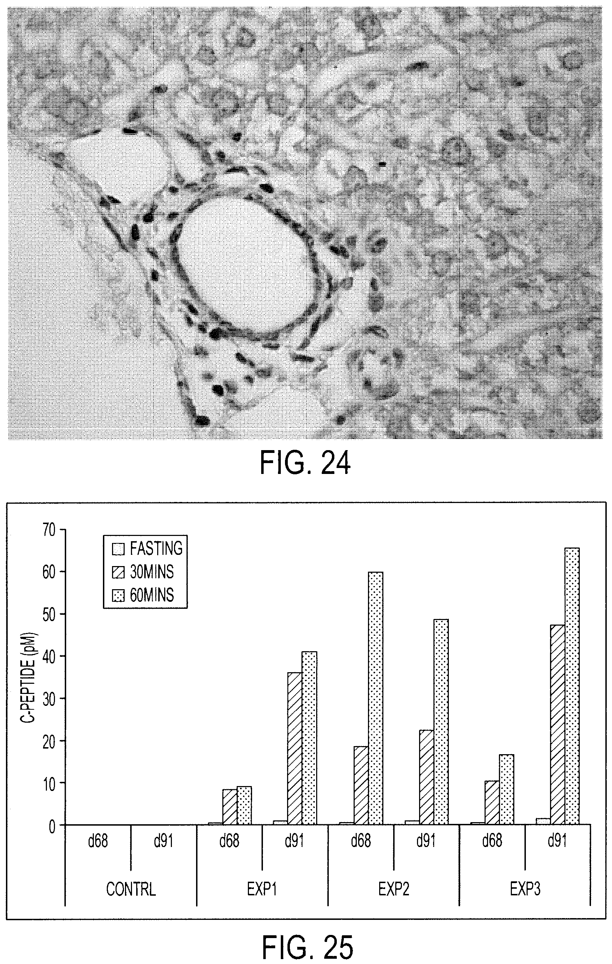

**Figure 24****.** Multipotentiality of the biliary tree setm cells as indicated by transplantation *in vivo:* Biliary Tree Fate. Biliary tree stem cells were maintained in culture under self-replication conditions (Kubota's Medium or its equivalent and culture plastic) and then were injected into the livers of immunocompromised, adult mice with quiescent livers (that is, no liver injury was induced). Liver sections prepared from the mice were then analyzed by immunohistochemistry for human specific markers indicative of cholangiocytes. Dako's anti-human CK7, a marker of cholangiocytes, was found on an average of 12.7 ± 5.5% of all cholangiocytes. In comparing the evidence for human cholangiocytes in small versus large bile ducts, it was found that ∼ 14.92 ± 5.9% cells in the large bile ducts and ∼ 5.02 ± 1.95% of the cells in small bile ducts were positive for human CK7.
**Figure 25****.** Multipotentiality of the biliary tree setm cells as indicated by transplantation *in vivo*: Pancreatic Fate. Biliary tree stem cells were maintained in culture under self-replication conditions (Kubota's Medium or its equivalent and culture plastic) and then were injected into the epididymal fat pads (EFP) of male Balb/C Rag2^{-/-}/Il2rg^{-/-} mice. Each mouse was injected with 200-400 neoislets, cell aggregates of Biliary Tree Stem Cells lineage restricted for 7-14 days towards a pancreatic islet fate in the 3-D differentiation conditions of an HDM-P plus a hydrogel containing hyaluronan, type IV collagen and laminin. Each neoislet was comprised of more than 1000 cells. The control mice were transplanted with Matrigel without cells. The mice were monitored daily for glucose levels and were hyperglycemic (600- 750 mg/dl) for about 3 months. By 3 months, the glucose levels in the transplanted mice had dropped to levels that were less than half that in the controls. Glucose tolerance tests were performed at postoperative day 68 and 91 and showed significant blood levels of human C-peptide in experimental mice, and these levels were regulatable by glucose.

### DETAILED DESCRIPTION OF THE INVENTION

The present invention stems from the heretofore unexpected discovery of a multipotent stem or progenitor cell, and cell populations comprising such multipotent stem or progenitor cells, found within the biliary tree and having the capacity to differentiate into multiple endodermal lineages, including liver, pancreas, and biliary tree **(****Figure 1****).** In the interest of clarity for this application, the term "Biliary Tree Stem Cell" will be used herein to refer to the disclosed mammalian multipotent stem or progenitor cell, cell populations comprising such cells, and cells populations enriched for such cells.

The multipotent Biliary Tree Stem Cells can be isolated from any portion of the biliary tree tissue but are found at especially high numbers in the peribiliary glands and at the branching points of the tree, which are indicated by stars on the schematic image of **Figure 2****.** The stars found on the smallest branching terminals within the liver refer to the canals of Hering, the stem cell niche for intrahepatic hepatic stem cells. The Biliary Tree Stem Cells are precursors to the intrahepatic hepatic stem cells.

### Cell Sourcing-Biliary tree

The biliary tree or extrahepatic biliary tree system is comprised of a series of ducts connecting the duodenum to the liver and to the pancreas and including the gallbladder (se schematic in **Figure 2**). Throughout the biliary tree, within the duct walls, are peribiliary glands **(****Figures 3-11****),** which may be found in particularly high numbers at the branching sites such as the hilum, common hepatic duct, cystic duct, common duct, common hepato-pancreatic duct and gallbaldder. Fluids from liver or from ventral pancreas are emptied into the duodenum via the Papilla of Vater. This entire group of structures, including the gallbladder, is referred to herein as the biliary tree.

All of the biliary tree *(e.g.,* the hilum, common hepatic duct, cystic duct, common duct, common hepato-pancreatic duct and gallbaldder) have walls composed of dense, fibrous connective tissue and a lumen lined by a layer of highly columnar biliary epithelium supported by a conventional basement membrane. Smooth muscle cells are dispersed along the ducts particularly near the Papilla of Vater. Blood vessels, nerve fibers and some lymphoid cells are found occasionally in the duct walls. Peribliary glands are found along the entire length of the biliary tree and in especially high numbers in the common hepato-pancreatic duct and the hilum, common hepatic duct, cystic duct, common duct and gallbladder. **Figures 3-7****.**

The gallbladder has different features **(****Figures 9****,** **10****).** The lumen is lined by columnar epithelia, a proper muscle layer and subserosal connective tissue. No peribiliary glands are present in the gallbladder **(****Figures 3****,** **9****).** However, the gallbladder does have cells with overlapping phenotypes to those of the biliary tree stem cell populations found within the peribiliary glands are transit amplifying cells and/or committed progenitors.

The cells of the disclosure may be isolated from biliary tree tissue of any stage of development. Thus, the instant invention may be practiced with fetal, neonatal, pediatric or adult tissue, including tissue from recently deceased individuals (preferably, within 10 hours, but the inventive cells remain viable for isolation of up to 72 hours post mortem). In fact, the biliary tree tissue is unique in that it is readily available from fetal, pediatric and adult donors. Furthermore, the present invention may be practiced with tissue from liver and pancreas organs obtained for, but then rejected for, transplantation, or from biopsy tissue, from resection tissue.

As well, the teachings herein are not limited or applicable to any one mammalian species. In fact, it should be understood that the examples provided herein are merely exemplary and should not be construed as limiting. The instant invention, in this way, is not limited by its mammalian source for biliary tree tissue. Mammals from which the Biliary Tree Stem Cells may be derived include, but are not limited to, humans, rodents (e.g., rats, mice, hamsters), rabbits, cows, horses, pigs, sheep, dogs and cats. Preferably, the Biliary Tree Stem Cells are derived from humans for use in humans.

As noted, the class of Biliary Tree Stem Cells can be differentiated into multiple endodermal fates. Indeed, the Biliary Tree Stem Cells may be induced to differentiate into mature cell types of several endodermal lineages including liver, biliary tree and pancreas. **Figures 17-25****.**

Samples of biliary tree tissue can be dissected surgically from livers or pancreas obtained for and then rejected for transplant due to reasons such as steatosis; anatomical abnormality, or major vascular disease; or they can be obtained from resection material. They can be from gallbladders removed for various reasons. The biliary tree tissue can be removed from the connective tissue of the abdomen. The tissue is then divided into segments and processed further. Segments that are especially rich in the Biliary Tree Stem Cells include: the hilum, common hepatic duct, cystic duct, common duct, common hepato-pancreatic duct and gallbladder. Each part can be further dissected into pieces cutting along the longitudinal diameter.

The Biliary Tree Stem Cells have been shown to give rise to multiple endodermal fates including liver, biliary tree and pancreas cells **(****Figures 17-25****).** The Biliary Tree Stem Cells express telomerase protein; low to moderate levels of pluripotency genes (Nanog, SOX2, KLF4 and OCT4-**Figure 8****);** classic endodermal transcription factors (*e*.*g*., SOX17, SOX 9, FOXA2, HNF6, PROX1, HNF3B (hepatocyte nuclear factor-3B (a.k.a. FOXA2), SALL4 (Sal-like protein 4), PDX 1 and NGN3); endodermal surface markers (*e.g*., CD326/Epithelial cell adhesion molecule or EpCAM; CD56/Neuronal cell adhesion molecule or NCAM); CXCR4; and several stem/progenitor genes *(e.g.,* CD44H-- hyaluronan receptors, and CD133, also called prominin). **Figures 7****,** **8****,** **9****,** **13****.**

Furthermore, ostensibly due to their multipotency, the Biliary Tree Stem Cells express low and variable levels of early lineage markers of the liver *(e.g.,* HNF6, HES1, FOXA2, and variably albumin), bile duct *(e.g.,* cytokeratin 19), and endocrine pancreas *(e.g.,* PDX1, NGN3, SALL4, insulin). **Figures 7****,** **22**

Notably, the Biliary Tree Stem Cells express the transcription factors PDX1 and NGN3 **(****Figure 3****,** **4****,** **7****,** **11****),** known to be essential for development of the pancreas and the endocrine pancreas, respectively. However, the Biliary Tree Stem Cells do not express or only weakly express mature markers of cholangiocytes (*e.g*., secretin receptor, aquaporins), hepatocytes (*e.g*., albumin, tyrosine aminotransferase or TAT, transferrin, "late" P450s such as P450-3A4) or islet cells *(e.g.,* glucagon, somatostatin, amylase or high levels of insulin) **(****Figures 3****,** **7****,** **13****).** They do not express at all markers for mesenchymal cells *(e.g.,* CD 146, desmin), endothelial cells *(e.g.,* VEGF receptor, CD31, Van Willebrand Factor) or hemopoietic cells *(e.g.,* CD45). The pattern of expression of the antigens is stable throughout the life of the cultures as long as they are maintained under self-replication conditions consisting of Kubota's Medium or its equivalent and with a substratum of culture plastic or hyaluronans.

These expression patterns can be determined using endpoint and quantitative (q)-RT-PCR assays and by immunohistochemistry of tissue *in vivo,* of freshly isolated cells, or of cultured cells. The co-expression in cells within the same peribiliary gland of multiple markers of endodermal stem/progenitors (*e.g*., SOX 9, SOX17, PDX1, NGN3, FOXA2) is a unique and surprising feature that is distinctive from the findings with respect to embryonic stem (ES) cells undergoing lineage restriction to pancreas and in which these transcription factors are observed sequentially, but not all at the same time. Furthermore, the expression of these transcription factors is absent in mature biliary cells at the lumenal surface of the bile ducts.

The Biliary Tree Stem Cells, as explained above, are progenitors to mature endodermal tissues and share markers with cells of the multiple tissues to which they can give rise including liver, biliary tree, and pancreas.

In addition to their relative expression levels, the cellular localization of telomerase and of the transcription factors (*e.g*., SOX 17, PDX1) expressed within the Biliary Tree Stem Cells changes from a nuclear localization to a perinuclear/cytoplasmic localization during maturation (See **Figure 7** and also **Figures 14-16****).** Within a single peribiliary gland, one observes some cells with nuclear and some cells with perinuclear localization or with no expression of the given marker **(****Figures 4****,** **6****,** **7****,** **14****).** Without being held to or bound by theory, intranuclear localization is associated with more primitive cells than those with perinuclear localization of the transcription factor(s). The latter cells are assumed to be transitioning into a later lineage stage. Alternatively, the perinuclear localization could indicate that the factor(s) is in an inactive storage form that can be translocated to the nucleus under appropriate regenerative demands.

The instant invention provides techniques for the isolation of Biliary Tree Stem Cells. The stem cell populations from human biliary tree tissue were identified by culture selection technologies but can be isolated also by immunoselection technologies (*e.g*., flow cytometry, panning, magnetic bead isolation). Alternatively, or in addition to immunoselection, the Biliary Tree Stem Cells may be isolated by virtue of tissue culturing conditions. For example, cell suspensions prepared from the biliary tree tissue may be plated onto plastic or hyaluronans. The plastic is coated optionally with collagen IV, collagen III, laminin, hyaluronans, other matrix components found in embryonic/fetal tissues, or combinations thereof.

The medium used for culture selection, serum-free Kubota's Medium or its equivalent, is strongly selective for the survival and proliferation of the Biliary Tree Stem Cells and their partner mesenchymal cells, angioblasts and stellate cell precursors, but is not selective for mature cells of the biliary tree. The essence of this medium is that it is any basal medium containing no copper, low calcium (<0.5mM), insulin, transferrin/Fe, free fatty acids bound to purified albumin and, optionally, also high density lipoprotein.

Kubota's Medium or its equivalent is serum-free and contains only purified and defined mix of hormones, growth factors, and nutrients. More specifically, the medium is comprised of a serum-free basal medium (*e.g*., RPMI 1640) containing no copper, low calcium (< 0.5 mM) and supplemented with insulin (5 µg/ml), transferrin/Fe (5µg/ml), high density lipoprotein (10 µg/ml), selenium (10⁻¹⁰ M), zinc (10⁻¹² M), nicotinamide (5 µg/ml), and a mixture of free fatty acids bound to a form of purified albumin. The detailed methods for the preparation of this media have been published elsewhere, *e*.*g*., Kubota H, Reid LM, Proceedings of the National Academy of Sciences (USA) 2000; 97:12132-12137.

These conditions yield colonies of Biliary Tree Stem Cells that grow rapidly for weeks (more than 8 weeks), with proliferation rates estimated at a division every 36-40 hours. **Figures 11-13****.** The cultures are able to remain stably as stem/progenitors for more than 8 weeks **(****Figure 13****),** evidence that they are undergoing self-replication. This evidence for self-replication is further supported by the finding of weak to moderate levels of expression of pluripotency genes. **Figure 8****.** The number of colonies obtained is highest in cultures from the portions of the biliary trees having large numbers of peribiliary glands and intermediate in those from sites other than the branching points of the biliary tree or from gall bladder with no peribiliary glands.

### Lineage Restriction and Differentiation to Adult Fates

Consistent with the multipotency of the Biliary Tree Stem Cells, if a colony is maintained for weeks (e.g., over a month in culture) under self-replication conditions and then the medium changed to a serum-free, hormonally defined medium (HDM) tailored for a specific adult cell type, the cells will partially differentiate towards the designated adult cell type **(****Figures 17-19****).** In an HDM for liver (HDM-L), 20-30 % of the cells acquired expression of cytokeratin 8 and 18 and albumin, whereas in an HDM for cholangiocytes (HDM-C), over half of them matured to cells expressing secretin receptor and CFTR. **Figure 17****.** The level of expression of human albumin in the cultures in HDM-L and the expression of secretin receptor in those in HDM-C were significantly higher than those in Kubota's Medium or its equivalent (self-replication conditions) in assays using quantitative RT-PCR **(****Figure 19****).**

Partial lineage restriction of the cells towards a pancreatic islet fate occurred if the Biliary Tree Stem Cells were cultured in an HDM for pancreas (HDM-P). **Figure 18****.** The differentiation occurred primarily at the edges of the colonies, sites at which aggregates of cells formed and inside which human C-peptide, PDX1, and insulin were found. More than half of the colonies of cells acquired the ability to produce human C-peptide **(****Figure 18h and 18g****),** indicative of human insulin synthesis, and this C-peptide synthesis was regulatable by glucose levels **(****Figure 18h****).** The level of human insulin produced was significantly higher in the cultures in HDM-P as compared with those remaining as stem cells under self-replications conditions. **(****Figure 19****).**

The extent of multipotentiality was demonstrated more dramatically if the cells were placed into distinct 3-D differentiation conditions comprised of the specific HDM (HDM-L, HDM-C, HDM-P) in combination with embedding the cells in hydrogels containing specific extracellular matrix components tailored for the adult cell type of interest **(****Figure 20****).** The cells rapidly differentiated (*e.g*., 7-10 days) to specific adult cell types - yielding cords of liver cells in HDM-L in combination with hydrogels of hyaluronans containing type IV collagen and laminin; branching bile ducts, i.e. biliary tree, in HDM-C in combination with hydrogels of hyaluronans containing type I collagen; or pancreatic neoislets (endocrine pancreas) if in HDM-P and in hydrogels containing type IV collagen and laminin.

Further evidence for the differentiation under the 3-D culture conditions was found for Biliary Tree Stem Cells under self-replication conditions **(****Figure 21****, a and b)** versus conditions tailored for hepatocytes **(****Figure 21, c and d****)** and then characterized by transmission electron microscopy. Transmission electron microscopy of Biliary Tree Stem Cells under self replication conditions (a-b) vs hepatocytes (c-d). Under differentiation conditions, large and polygonal cells were present, and cell nuclei display one or more nucleoli. Adjacent cells form well-defined bile canaliculi (arrows). Bile canaliculi are closed by junctional complexes *(arrowheads).* Few microvilli are present in the lumen. The bar on the figures is equal to 2 *µ*m.

Proof that the differentiation conditions resulted in functional adult cells is provided in **Figure 22** containing quantitative RT-PCR data from cultures either under self-replication conditions yielding biliary stem/progenitors (BP) versus under the 3-D conditions for liver (B-L), biliary tree/cholangiocytes (B-C), or pancreas (B-P). The top row indicates the dramatic increase in expression of classic liver genes---HNF4, AFP, albumin, tyrosine aminotransferase (TAT), transferrin (TF), and P450-3A4---in the cultures under conditions for liver but not under those for the stem/progenitors or for biliary tree or pancreas. By contrast, those plated under conditions for pancreas (second row) showed very dramatic increases in gene expression for PDX-1, ISL-1, NGN3, insulin, glucagon (GCG) under the conditions for pancreas (B-P) but not those for stem/progenitors or liver or biliary tree. Those plated under conditions for biliary tree demonstrated increases for expression of GGT1, AE2, CFTR. The cultures of Biliary Tree Stem Cells transferred again under the self-replication conditions maintained high levels of EpCAM and low levels of all the adult-specific genes.

This multipotentiality is demonstrated also when the cells are transplanted *in vivo* **(****Figures 23-25****).** Human Biliary Tree Stem Cells were transplanted into immunocompromised mice and then tissues evaluated later for presence of engrafted mature human cells. Even in mice with quiescent livers, that is not subjected to liver injuries, the cells were able to engraft and mature into significant numbers of hepatocytes **(****Figure 23****)** and cholangiocytes **(****Figure 24****).** Moreover, the mice transplanted with the cells driven to a pancreatic fate were subjected to drugs that made them diabetic, and the transplanted cells were able to rescue them from the diabetic condition, and these cells proved to be responsive to glucose levels in terms of production of human C-peptide **(****Figure 25****).**

Although multiple precursors have been identified and shown to differentiate into mature liver cells, the Biliary Tree Stem Cells identified in the present application, and present in fetal, neonate, pediatric and adult tissues are the first stem cells and cell populations identified to date that can be isolated from adult tissues and proven able to differentiate to mature pancreatic cell types. These cells are also the first identified that can be used immediately in clinical programs for diabetes because of their demonstrated ability to be able to differentiate into pancreatic islet cells, their lack of tumorigenic potential (as exists for ES cells or cells that are transfected with genes critical for differentiation to pancreas), and lack of immunogenicity.

The Biliary Tree Stem Cells are the natural precursors to pancreas and can differentiate easily and rapidly (within a few days in cultures) to pancreatic fates merely by using specific micro-environmental conditions. Moreover, the transition to the clinic is also facilitated by the fact that the micro-environmental conditions needed are available in GMP forms and now are part of extant clinical therapies. Hence, at least one clinical application is the use of the Biliary Tree Stem Cells described herein for the complete or partial repopulation, rescue, support, repair, replacement or introduction of pancreatic beta-like cells for treatment of diabetes **(****Figure 25****),** or for forms of liver failure, insufficiency, or degeneration **(****Figures 23** **and** **24****).**

It should be apparent to one of ordinary skill in the art in view of the disclosure herein that the Biliary Tree Stem Cells have numerous applications in clinical therapy and treatment. The acquisition of the Biliary Tree Stem Cells can be a relatively non-invasive procedure and relatively harmless to the patient. The ability to propagate these biliary stem cells *in vitro* facilitates the ability to obtain sufficient cells for clinical programs (*e.g*., 10⁶-10⁹ cells). The quantity of stem cells needed for cell therapy programs is readily generated within a few weeks after the cells are procured, processed and cultured as indicated in **Figure 13****.**

The Biliary Tree Stem Cells provided for therapy can be prepared from a given donor and then given back to the same person, constituting autologous therapy with no immunological problems in terms of cell rejection, as cells and recipient are preferably genetically identical. Alternatively, the Biliary Tree Stem Cells or cell populations provided for therapy can be prepared from a given donor and then given to another person, constituting allogeneic therapy as the Biliary Tree Stem Cells are non-immunogenic or minimally immunogenic. Finally, the Biliary Tree Stem Cells should be relatively risk-free in animal experimentation and in cultures with regard to oncogenic potential. In all *in vivo* studies to date, there has been no evidence at all of tumorigenic potential.

The Biliary Tree Stem Cells or cell populations are used for the *in vitro* production of target endodermal cells and target endodermal tissue (*e.g*., pancreas, liver, bile duct). Accordingly, methods of producing the "target" cells and tissues from the Biliary Tree Stem Cells or cell populations are disclosed, which methods comprise the isolation of Biliary Tree Stem Cells, their incubation under conditions that drive their differentiation into the target tissue or cells of that tissue, and introduction of the cells into a patient in need thereof.

As noted above and demonstrated in **Figures 14-16****,** for partial differentiation of the Biliary Tree Stem Cells towards a specific adult fate, according to the invention, one can use a defined medium (also called a Hormonally Defined Medium or HDM) containing a specific mix of nutrients, hormones and growth factors, ideally being serum-free, and tailored to drive the cells to the adult fate of interest. Representative HDM for hepatocytes, cholangiocytes versus pancreatic islets are given below. The HDM alone elicits some lineage restriction towards the desired adult fate but does not elicit full differentiation. Full differentiation occurs after transplantation *in vivo* or, if the cells are *ex vivo,* further requires use of a specific HDM in combination with a mix of extracellular matrix components, the exact composition of the mix being unique to the adult cell type desired, and the cells must be established in a three-dimensional format as described herein. **Figures 17-19****.**

Particularly preferable forms of application for *in vivo* differentiation of the Biliary Tree Stem Cells or cell populations are injection, infusion or implantation by grafting method of the Biliary Tree Stem Cells or cell populations into one area of the body, in order to allow for the Biliary Tree Stem Cells to differentiate there, by direct contact with cells of the target lineage or infusion of the Biliary Tree Stem Cells or cell populations to allow for the Biliary Tree Stem Cells or cell populations to reach the target tissue.. For injection or infusion, the Biliary Tree Stem Cells can be administered in a medium with which they are compatible, such as Kubota's Medium (or equivalent) or one of the HDM, or in a graft or implant under conditions comprised of matrix components with which they are compatible.

### Therapeutic applications

For the therapeutic use of the target cells obtainable from the Biliary Tree Stem Cells or cell populations, a number of concepts are available *(see* Science 287: 1442-1446, 2000). Examples of relevant indications in this connection are: inborn errors of metabolism, liver failure, cirrhosis of the liver, pancreatic insufficiency, and diabetes.

The inventive Biliary Tree Stem Cells or cell populations can be introduced directly into or onto the organ to be reconstituted, renewed or repaired. The introduction can be carried out as cell suspensions, as grafts comprised of the Biliary Tree Stem Cells or cell populations incorporated into a mix of extracellular matrix components along with the HDM or as other types of scaffolds (e.g., microcarriers, polylactides) or as infusions. The scaffolds are preferably biodegradable, so that they "disappear" from the body, while the newly introduced cells or cell populations grow together with the existing cells. Cells that may be reconstituted, rescued, supported, repaired, replaced or introduced in this manner, preferably by autologous transplant, include islet cells or other pancreas cells, hepatocytes or other liver cells, and cholangiocytes or other biliary tree cells. Reconstitution, rescue, repair, support, replacement, or introduction may follow partial surgical resection of an organ for repair after trauma or for supportive use, for example, in the case of lacking or insufficient organ function.

The Biliary Tree Stem Cells or cell populations and target cells obtained from them can further be bound to implantable materials, in order to increase biocompatibility. Therefore, also implantable materials, when coated with the Biliary Tree Stem Cells, are disclosed. The implantable materials can also be artificial and/or biological carrier or support materials, which contain the Biliary Tree Stem Cells or cell populations and/or target cells derived therefrom. In this regard, the carrier materials or support materials can be microcarriers, supports, containers or chambers for insertion or implantation into the human body.

For instance, a container with islet cells derived from the Biliary Tree Stem Cells or cell populations may be used for the production of a pharmaceutical construct for use as an artificial islet cell port chamber to supply insulin *in vivo.* Alternatively, an infusion or graft of the Biliary Tree Stem Cells or cell populations may be used to reconstitute, rescue, repair, support, replace, or introduce islets or islet cells *in vivo.* Similar constructs can be made with hepatocytes or cholangiocytes derived from the Biliary Tree Stem Cells.

The target cells or cell populations obtained from the Biliary Tree Stem Cells cells can in addition be used as cell cultures in bioreactors (e.g., external to the body), for example in order to carry out detoxification reactions or to produce substances generated normally *in vivo* by the target cells or tissues. This form of use is particularly relevant in the case of acute conditions, for example, in the case of acute liver failure as a bioartificial liver or for severe diabetes as a bioartificial endocrine pancreas.

Finally, the multipotent Biliary Tree Stem Cells or cell populations may be broadly applied in transgenic modification and therapy. The Biliary Tree Stem Cells, or cells or tissue differentiated therefrom, may be transfected with one or more genes. In this way, one or more genes, which are required to maintain the metabolism of certain organs, such as for example liver or pancreas, are restored and/or supported or reintroduced. For example, stem cells or hepatocytes can be transfected with the FAH (fumaroylacetoacetate hydrolase) gene. In a FAH-deficient mouse model, the intrasplenic injection of 1000 FAH-positive donor hepatocytes is sufficient to completely reconstitute the liver and fully compensate for the metabolic defect leading to cirrhosis of the liver. Overturf, K., M. Al-Dhalimy, C.-N. Ou, M. Finegold, and M. Grompe. American Journal of Pathology 151:1273-1280 (1997). Alternatively, the Biliary Tree Stem Cells or cell populations can be prepared from a given donor and delivered to another person, constituting allogeneic therapy, in order to restore, support, or introduce in the recipient one or more genes required to maintain the metabolism of certain organs, such as, for example, the liver or pancreas.

The following examples are illustrative of the invention, but the invention is by no means limited to these specific examples. A person of ordinary skill in the art will find in these examples but one means to implement the instant invention. Further, while the instant examples have been presented in the context of non-humans for experimental convenience, the methods and reagents described herein can be readily translated to human application(s) by one of ordinary skill in the art from the teachings disclosed below.

### EXAMPLES

### EXAMPLE I - Cell preparation

Enzymatic dissociation may be carried out in the presence of protease(s), such as collagenase(s), and/or nuclease(s), such as DNase. Methods of enzymatic dissociation of liver cells are described and practiced in the art. By way of example, methods for the isolation and identification of the hepatic progenitors have been described in, for example, USP No. 6,069,005 and USP Application Nos. 09/487,318; 10/135,700; and 10/387,547. Indeed, various procedures exist for preparation of cell suspensions. It is to be understood, therefore, that the scope of the present invention is not to be limited to a specific method of procuring whole tissue or preparing cell suspensions thereof.

### EXAMPLE II - 3D culture conditions

3-dimensional (3-D) gels may be formed by mixing the matrix components, the hormones, cytokines, growth factors, nutrients and the basal medium into hyaluronans that are liquid when not cross-linked and become gelled when cross-linked. The details of the preparation of these are published elsewhere, *e*.*g*., W. S. Turner, E. Schmelzer, R. McClelland et al., J Biomed Mater Res B Appl Biomater 82B (1), 156 (2006); and W. S. Turner, C. Seagle, J. A. Galanko et al., Stem Cells 26 (6), 1547 (2008). The cultures are typically maintained for 2-4 weeks or longer and then analyzed by histology, gene expression assays such as endpoint and quantitative RT-PCR, immunofluorescence and protein expression assays such as Western blots, and metabolomic footprinting.

The major components of the gel complexes are forms of chemically-modified hyaluronans. Carbylan-S (or, CMHA- S) is a carboxymethlated hyaluronan derivative that has been modified with multiple thiols for crosslinking. All materials are commercially available from, at least, Glycosan Biosciences (Salt Lake City, Utah).

Briefly, in one embodiment, the hyaluronan-matrices are prepared by dissolving hyaluronan as a dry reagent in Kubota's Medium to give a 2.0% solution (weight/volume). Laminin (Sigma, St. Louis, Mo) may then be added at a concentration of 1.5 mg/ml and type IV collagen (Becton Dickenson, Bedford, Ma) at a concentration of 6 mg/ml. Cross-linking occurs within hours at room temperature and with exposure to oxygen in the air or within minutes if exposed to a cross-linking reagent, *e.g*., PEGDA (polyethlylene glycol diacrylate). So, the solution should be kept chilled at 4 °C and contact with oxygen and/or with PEGDA avoided, if cross-linking is not desired.

Upon completion of the experiments, the cells may be recovered by digesting the hydrogels first with a mix of hyaluronidase (1 mg/ml), DNase (0.5 mg/ml), and dithiothreitol (40 mgs/ml) prepared in Kubota's Medium (without transferrin), followed by Liberase (0.5 mg/ml) prepared in Kubota's Medium without insulin and transferrin. The cells that are obtained in this manner are suitable for characterization by flow cytometry, RT-PCR, or immunohistochemistry.

### EXAMPLE III - Evidence of multipotency

After 7-30 days or longer of culturing Biliary Tree Stem Cells or cell populations under self-replication conditions (Kubota's Medium, or equivalent, in combination with culture plastic or hyaluronans), monolayer cultures of the remaining cells (*i.e.,* Biliary Tree Stem Cells) are transferred to a differentiation medium, wherein the stem cells undergo rapid shape changes and changes in gene expression within 48 hours. All the differentiation media consists of modifications to the medium used for self-replication (*e.g*., Kubota's Medium or equivalent) such that the hormonally defined medium will contain the components in the self-replication medium plus supplementation with calcium (≥0.5 mM), copper, bFGF; this is referred to as "modified medium". To achieve a specific adult cell type, the following are required:

Liver: lineage restriction to liver fates (*e.g*., hepatocytes) may be achieved by embedding the Biliary Tree Stem Cells into hydrogels of hyaluronans into which is mixed type IV collagen and laminin and with the "modified medium" further supplemented with glucagon, galactose, triiodothyroxine, epidermal growth factor (EGF) and hepatocyte growth factor (HGF). In one embodiment, the amount of ingredients are as follows: MKM supplemented with 7*µ*g/L glucagon, 2g/L galactose, 10⁻⁹ M triiodothyroxine 3, 10 ng/ml EGF and 20 ng/ml HGF.

Pancreatic tissue: lineage restriction to pancreatic fates (*e.g*., islets) may be achieved by embedding the Biliary Tree Stem Cells into a hydrogel of hyaluronans containing type IV collagen and laminin and with the "modified medium" further changed to remove hydrocortisone and to contain B27, ascorbic acid, cyclopamine, Retinoic acid and exendin 4. In one embodiment, the amount of ingredients are as follows: MKM modified to remove hydrocortisone and contain 2% B27, 0.1 mM ascorbic acid, 0.25 µM cyclopamine, 0.5µM Retinoic acid and 50ng/ml exendin 4.

Biliary Tree/Cholangiocytes: similarly, the Biliary Tree Stem Cells can be lineage restricted to biliary fates (e.g., cholangiocytes) by embedding the stem cells into hyaluronans mixed with type I collagen and in the "modified medium" further supplemented with vascular endothelial cell growth factor (VEGF) 165 and HGF. In one embodiment, the amount of ingredients are as follows: MKM further supplemented with 20 ng/ml vascular endothelial cell growth factor (VEGF) 165 and 10 ng/ml HGF.

### EXAMPLE IV - Transplantation

Transplantation of cells from solid organs via grafting protocols are preferable over injection or infusion for many applications, although either mode of delivery is feasible. It has been found that the hydrogel cultures described hereinabove provide good conditions for grafting. The cells can be suspended in the un-crosslinked hyaluronans, mixed with other matrix components in medium comprising basal medium plus hormones, growth factors and other soluble signals tailored for expansion and/or differentiation.

The cell populations used for the transplantation preferably comprise Biliary Tree Stem Cells or cell populations that are transplanted along with (or without) their native mesenchymal partners (*e.g*., angioblasts, stellate cells), derived from the target tissue (or other source) for the transplantation, and at ratios paralleling those found *in vivo.* Without being held to or bound by theory, it is believed that this combination of the stem cells and the partner mesenchymal cells provides the appropriate microenvironment for full maturation of tissues that are vascularized and able to function physiologically.

In one embodiment, for pancreatic grafts, implants, or injections, the cellular components of the grafts may consist of the Biliary Tree Stem Cells or cell populations co-seeded into the graft with human fetal or neonatal tissue-derived angioblasts (VEGFR⁺, CD117⁺ cells) and stellate cells (CD146⁺ cells) at an estimated ratio of those cell populations found *in vivo* in the tissue. The stem cell populations may be obtained from expanding cells in culture as described herein. The angioblasts and stellate cells may be immunoselected with magnetically activated cell sorting (MACS) system or flow cytometric sorts from freshly prepared cell suspensions of human fetal or neonatal pancreas tissue.

All of the biliary tree stem cell populations can be immunoselected for cells positive for a cell surface marker common to stem/progenitors (*e*.*g*., CD133, CD44H, EpCAM, NCAM). In one embodiment, the cells are incubated at 4 °C for 20 minutes at a concentration of 50 µl for 50 x 10⁶ total cells in 500 µl phosphate buffered saline (PBS) containing 0.5% bovine serum albumin and 2 mM EDTA with FITC-conjugated primary antibodies. The cells so labeled are reacted with magnetic beads using anti-FITC antibodies and then selected by Midi- or MiniMACS columns and separation units. All incubation and selection steps should be performed on ice with addition of 10% ACCUTASE (Innovative Cell Technologies, San Diego, CA) to prevent aggregation of cells. Alternatively, the cells can be labeled with a fluoroprobe-labeled antibody and the cells immunoselected using flow cytometer.

### EXAMPLE V - Media

All media were sterile-filtered (0.22-*µ*m filter) and kept in the dark at 4°C before use. Kubota's medium (KM) consists of any basal medium (*e.g*., RPMI 1640) with no copper, low calcium (<0.5 mM), 10⁻⁹ M selenium, 4.5 mM nicotinamide, 0.1 nM zinc sulfate, 10⁻⁸ M hydrocortisone, 5 µg/ml transferrin/Fe, 5 µg/ml insulin, a mixture of free fatty acids that are added bound to serum albumin (0.1%) and, optionally, 10 µg/ml high density lipoprotein.

### EXAMPLE VI - Differentiation conditions

Three dimensional cultures were established with hydrogels containing hyaluronans, other matrix molecules, hormones, growth factors, cytokines, all prepared in a mediuim. All of the hydrogels were made using modified Kubota's Medium (or equivalent) that was supplemented with calcium to achieve 0.6 mM concentration, 10⁻¹² M copper, and 10 µm of basic fibroblast growth factor (bFGF) (referred to as modified KM or MKM):

Liver cells: MKM-L with 7*µ*g/L glucagon, 2g/L galactose, 10⁻⁹ M tri-iodothyroxine 3, 10 ng/ml epidermal growth factor (EGF) and 20 ng/ml hepatocyte growth factor (HGF). The matrix scaffolds consisted of 60% type IV collagen and laminin and 40% hyaluronans.

Biliary Tree/Cholangiocytes: MKM-C supplemented with 20 ng/ml vascular endothelial cell growth factor (VEGF) 165 and 10 ng/ml HGF. The matrix scaffolds consisted of 60% type I collagen and 40% hyaluronans.

Pancreas, islets: MKM-P (without hydrocortisone) further supplemented with 2% B27, 0.1 mM ascorbic acid, 0.25 µM cyclopamine, 0.5µM RA and 50ng/ml exendin-4. The matrix scaffolds consisted of 60% type IV collagen and laminin and 40% hyaluronans.

### EXAMPLE VII - Cell isolation and phenotyping

Human biliary tissues were obtained from livers and pancreases obtained for and then rejected for transplantation into a patient. Cell suspensions of human biliary tissues were processed using RPMI 1640 supplemented with 0.1% bovine serum albumin, 1 nM selenium and antibiotics. Enzymatic processing buffer contained 300U/ml type IV collagenase and 0.3 mg/ml deoxyribonuclease at 32 °C with frequent agitation for 15-20 min. Enriched suspensions were pressed through a 75 gauge mesh and spun at 1200 RPM for 5 min before resuspension. Estimated cell viability by trypan blue exclusion was routinely higher than 95%.

Approximately 3x10⁵ cells were plated on a 10 cm tissue culture dish and in serum-free Kubota's Medium, which was replaced every 3 days. Colonies formed within 5-7 days and were observed for up to 3 months. Colonies were picked by hand at varying times using an inverted microscope.

Multipotent Biliary Tree Stem Cells were also isolated by immunoselection from freshly prepared cell suspensions based on positive expression of one or more cell surface markers common to stem/progenitors (CD133, CD44H, NCAM, EpCAM-- CD326) using magnetic bead immunoselection technologies with the Miltenyi Biotech MACS system (Bergisch Gladbach, Germany) following the manufacturer's instructions. Briefly, dissociated cells were incubated with EpCAM antibody bound to magnetic microbeads for 30 min at 4 °C, and were separated using magnetic column separation system from Miltenyi following the manufacturer's recommended procedures. Medium was replaced daily and collected medium was stored at -20 °C for further analysis.

For the fluorescent staining, cells were fixed with 4% paraformaldehyde (PFA) for 20 min at room temperature, rinsed with HBSS, blocked with 10% goat serum in HBSS for 2 h, and rinsed. Fixed cells were incubated with primary antibodies at 4 °C over night, washed, incubated for 1h with labeled isotype-specific secondary antibodies, washed, counterstained with 4',6-diamidino-2-phenylindole (DAPI) for visualization of cell nuclei.

For immunohistochemistry, tissues were fixed in 4% paraformaldehyde (PFA) overnight, stored in 70% ethanol, and subsequently embedded in paraffin and cut into 5 µm sections. Sections were deparaffinized, and the antigens were retrieved. Endogenous peroxidases were blocked by incubation for 30 min in 0.3% H₂O₂ solution. After blocking with 10% horse serum, primary antibody was applied at 4 °C over night; secondary antibody and ABC staining were performed using the RTU Vectastain kit (Vector Laboratories, Burlingame, CA). Vector Nova RED was used as substrate. Sections were dehydrated, fixed and embedded in Eukitt Mounting Media (Electron Microscopy Sciences, Hatfield, PA), and were analyzed using an inverted microscope.

For quantitative reverse-transcription polymerase chain reaction (RT-PCR) analysis, biliary tree tissue or cells from culture were lysed and total RNA extracted using RNeasy Plus Mini Kit (Qiagen GmbH, Valencia CA) as per the manufacturer's instructions. Reverse transcription was carried out with the SuperScript First-Strand Synthesis System for RT-PCR (Invitrogen, Carlsbad, CA). HotStarTaq Master Mix Kit (Qiagen) was used for PCR.

Cells were analyzed and sorted by a FACStar Plus cell sorter (BD Biosciences) equipped with dual Coherent 1-90 lasers. Fluorescence-conjugated antibodies were excited at 488 nm, and their fluorescence emission was detected by standard filters.

**Abbreviations: AE2,** anion exchanger 2; **AFP,** *α*-fetoprotein; **ALB,** albumin; **ASMA,** alpha-smooth muscle actin; **ASBT,** Apical sodium-dependent bile acid transporter; **bFGF,** basic fibroblast growth factor; **CD,** common determinant; **CD133,** prominin; **CD146,** Mel-CAM; **CD31,** PECAM; **CD44H,** hyaluronan receptor; **CD45,** common leucocyte antigen; **CFTR,** cystic fibrosis transmembrane conductance regulator; **CK,** cytokeratin; **CXCR4,** CXC-chemokine receptor 4; **CYP450,** Cytochrome P450; **EGF,** epidermal growth factor; **EpCAM,** epithelial cell adhesion molecule; **FOXa2,** forkhead box a2; **GGT,** gamma glutamyl transpeptidase; **HDM,** hormonally defined medium, one tailored for a specific cell type; **HDM-L,** hormonally defined medium for liver; **HDM-C,** hormonally defined medium for cholangiocytes (biliary tree); **HDM-P,** hormonally defined medium for pancreas; **HGF,** hepatocyte growth factor; **HNF,** hepatocyte nuclear factor; **KM,** Kubota's Medium, a serum-free medium designed for stem/progenitors; **NCAM,** neural cell adhesion molecule; **NGN3,** neurogenin 3; **PDX1,** Pancreatic and duodenal homeobox 1; **PROX1,** Prospero homeobox protein 1; **SALL4,** Sal-like protein 4; **SOX,** Sry-related HMG box; **SR,** secretin receptor; **VEGF,** vascular endothelial growth factor.

## Claims

1. A method of obtaining mammalian multipotent stem/progenitor cells capable of differentiating into multiple endodermal lineages, wherein the cells are obtained from biliary tree tissue of a mammal, the method comprising obtaining biliary tree tissue, and sequentially, and in any order, or substantially simultaneously obtaining cells that are positive for:
(i) at least one stem cell surface marker selected from EpCAM, N-CAM, CXCR4, CD44H, or CD133;
(ii) at least one marker indicative of liver cell lineage stages selected from HNF6, HES1, CK19, albumin, or AFP; and
(iii) at least one marker indicative of pancreatic cell lineage stages selected from PDX1, PROX1, NGN3, or insulin.

2. The method of Claim 1, in which the mammalian multipotent stem/progenitor cells are obtained via immunoselection technology and/or selective culture conditions, preferably via immunoselection technology comprising panning, magnetic bead selection, flow cytometry, or combinations thereof, more preferably via flow cytometry.

3. The method of Claim 2, in which the selective culture condition comprises plating the cells onto plastic, onto hyaluronans or onto plastic optionally coated with collagen IV, collagen III, laminin, hyaluronans, other matrix components from embryonic, fetal, neonatal tissues or combinations thereof.

4. The method of Claim 2, further comprising incubating the cells in serum-free medium comprising a basal medium with low calcium, no copper, and supplemented with insulin, transferrin/Fe, selenium, zinc, free fatty acids bound to serum albumin and, optionally, high density lipoprotein.

5. The method of Claim 4, in which the basal medium comprises RPMI 1640.

6. The method of Claim 4, in which the basal medium comprises less than 0.5 mM calcium.

7. The method of Claim 2 in which the cells are cultured for 7-21 days or longer.

8. The method of Claim 1 in which the biliary tree tissue comprises hilum, common hepatic duct, cystic duct, common duct, common hepato-pancreatic duct and gallbladder, or combinations thereof.

9. The method of Claim 1 in which the biliary tree tissue is derived from sites at which the biliary tree branches or contains high numbers of peribiliary glands.

## Patentansprüche

1. Verfahren zum Erhalt von multipotenten Stamm-/Progenitor-Säugetierzellen, die in der Lage sind, in vielfache endoderme Zelllinien zu differenzieren, wobei die Zellen aus Gallengangsgewebe eines Säugetiers erhalten werden, wobei das Verfahren umfasst: Erhalten von Gallengangsgewebe, und sequentiell, und in jeglicher Reihenfolge, oder im Wesentlichen simultan, Erhalten von Zellen, die positiv sind für:
(i) Wenigstens einen Stammzelloberflächenmarker, ausgewählt aus EpCAM, N-CAM, CXCR4, CD44H oder CD133;
(ii) wenigstens einen Marker, der auf Leberzelllinienstadien hinweist, ausgewählt aus HNF6, HESI, CK19, Albumin oder AFP; und
(iii) wenigstens einen Marker, der auf Pankreaszelllinienstadien hinweist, ausgewählt aus PDX1, PROX1, NGN3 oder Insulin.

2. Verfahren nach Anspruch 1, bei dem die multipotenten Stamm-/Progenitor-Säugetierzellen mittels Immunselektionstechnologie und/oder selektiven Kulturbedingungen erhalten werden, bevorzugt über Immunselektionstechnologie, umfassend ein Panning, Selektion mittels magnetischer Beads, Durchflusszytometrie oder Kombinationen davon, bevorzugter mittels Durchflusszytometrie.

3. Verfahren nach Anspruch 2, in dem die selektive Kulturbedingung das Ausplattieren der Zellen auf Plastik, auf Hyaluronane oder auf Plastik umfasst, das wahlweise mit Kollagen IV, Kollagen III, Laminin, Hyaluronanen, anderen Matrixbestandteilen aus Embryonen-, Fötal-, Neonatal-Geweben oder Kombinationen davon, beschichtet ist.

4. Verfahren nach Anspruch 2, weiterhin umfassend ein Inkubieren der Zellen in serumfreiem Medium, umfassend ein basales Medium mit niedrigem Kalziumgehalt, keinem Kupfer, und das mit Insulin, Transferrin/Fe, Selen, Zink, freien an Serumalbumin-gebundenen Fettsäuren und, wahlweise, Lipoprotein hoher Dichte supplementiert ist.

5. Verfahren nach Anspruch 4, in dem das basale Medium RPMI 1640 umfasst.

6. Verfahren nach Anspruch 4, in dem das basale Medium weniger als 0,5 mM Kalzium umfasst.

7. Verfahren nach Anspruch 2, in dem die Zellen für 7-21 Tage oder länger kultiviert werden.

8. Verfahren nach Anspruch 1, in dem das Gallengangsgewebe den Hilus, den allgemeinen Gallengang, den Gallenblasenausführungsgang, den Hauptgallengang, den allgemeinen Leber-Pankreasgang und die Gallenblase, oder Kombinationen davon umfasst.

9. Verfahren nach Anspruch 1, in dem das Gallengangsgewebe von Stellen stammt, an denen sich der Gallengang verzweigt oder hohe Anzahlen an peribiliären Drüsen enthält.

## Revendications

1. Méthode d'obtention de cellules souches/parentes pluripotentes de mammifère capables de se différencier en plusieurs lignées endodermiques, dans laquelle les cellules sont obtenues à partir de tissu d'arbre biliaire d'un mammifère, la méthode comprenant l'obtention de tissu d'arbre biliaire, et séquentiellement, et dans n'importe quel ordre, ou sensiblement simultanément obtenir des cellules positives pour :
(i) au moins un marqueur de surface de cellules souches choisi parmi EpCAM, N-CAM, CXCR4, CD44H ou CD133 ;
(ii) au moins un marqueur indiquant des stades de lignée de cellules hépatiques choisis parmi HNF6, HES1, CK19, l'albumine ou AFP ; et
(iii) au moins un marqueur indiquant les stades de la lignée cellulaire pancréatique choisis parmi PDX1, PROX1, NGN3 ou l'insuline.

2. Méthode selon la revendication 1, dans laquelle les cellules souches/parentes pluripotentes de mammifère sont obtenues via la technologie d'immunosélection et/ou des conditions de culture sélectives, de préférence via la technologie d'immunosélection comprenant le panoramique, la sélection de billes magnétiques, la cytométrie en flux, ou leurs combinaisons, plus préférablement via la cytométrie en flux.

3. Méthode selon la revendication 2, dans laquelle la condition de culture sélective comprend le placage des cellules sur du plastique, sur des hyaluronanes ou sur du plastique éventuellement recouvert de collagène IV, de collagène III, de laminine, de hyaluronanes, d'autres composants de matrice issus de tissus embryonnaires, fœtaux, néonatals ou leurs combinaisons.

4. Méthode selon la revendication 2, comprenant en outre l'incubation des cellules dans un milieu sans sérum comprenant un milieu basal à faible teneur en calcium, sans cuivre et complété par de l'insuline, de la transferrine/Fe, du sélénium, du zinc, des acides gras libres liés à l'albumine sérique et, facultativement, lipoprotéines de haute densité.

5. Méthode selon la revendication 4, dans laquelle le milieu basal comprend du RPMI 1640.

6. Méthode selon la revendication 4, dans laquelle le milieu basal comprend moins de 0,5 mm de calcium.

7. Méthode selon la revendication 2, dans laquelle les cellules sont cultivées pendant 7 à 21 jours ou plus.

8. Méthode selon la revendication 1, dans laquelle le tissu de l'arbre biliaire comprend le hile, le conduit hépatique commun, le conduit cystique, le conduit commun, le conduit hépato-pancréatique commun et la vésicule biliaire, ou leurs combinaisons.

9. Méthode selon la revendication 1, dans laquelle le tissu de l'arbre biliaire est dérivé de sites où l'arbre biliaire se ramifie ou contient un grand nombre de glandes péribiliaires.
